(19)

**Europäisches Patentamt**
**European Patent Office**
**Office européen des brevets**

(11)　　**EP 2 633 077 B1**

(12)　　**EUROPEAN PATENT SPECIFICATION**

(45) Date of publication and mention
of the grant of the patent:
**07.06.2017 Bulletin 2017/23**

(21) Application number: **11791614.8**

(22) Date of filing: **26.10.2011**

(51) Int Cl.:
***C12Q 1/68*** (2006.01)

(86) International application number:
**PCT/GB2011/052082**

(87) International publication number:
**WO 2012/056236 (03.05.2012 Gazette 2012/18)**

(54) **ANALYTICAL METHODS AND ARRAYS FOR USE IN THE IDENTIFICATION OF AGENTS INDUCING SENSITIZATION IN HUMAN SKIN**

ANALYTISCHE VERFAHREN UND ANORDNUNGEN ZUR VERWENDUNG BEI DER IDENTIFIZIERUNG VON SENSIBILISIERUNGSINDUZIERENDEN WIRKSTOFFEN AUF DER MENSCHLICHEN HAUT

PROCÉDÉS D'ANALYSE ET PUCES UTILISABLES POUR L'IDENTIFICATION D'AGENTS INDUISANT UNE SENSIBILISATION DE LA PEAU HUMAINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.10.2010 GB 201018014**

(43) Date of publication of application:
**04.09.2013 Bulletin 2013/36**

(73) Proprietor: **SenzaGen AB**
**223 81 Lund (SE)**

(72) Inventors:
• **LINDSTEDT, Malin**
**24791 Södra Sandby (SE)**
• **BORREBAECK, Carl A K**
**S-22363 Lund (SE)**
• **JOHANSSON, Henrik**
**21158 Malmö (SE)**
• **ALBREKT, Ann-Sofie**
**S-26020 Teckomatorp (SE)**

(74) Representative: **Potter Clarkson LLP**
**The Belgrave Centre**
**Talbot Street**
**Nottingham NG1 5GG (GB)**

(56) References cited:
**WO-A1-2009/148669　WO-A1-2010/031799**

• **S. SZAMEIT ET AL: "Gene expression studies in cultured dendritic cells: new indicators for the discrimination of skin sensitizers and irritants in vitro", CLINICAL & EXPERIMENTAL ALLERGY, vol. 39, no. 6, 1 June 2009 (2009-06-01), pages 856-868, XP55002859, ISSN: 0954-7894, DOI: 10.1111/j.1365-2222.2009.03222.x**
• **CLUZEL-TAILHARDAT ET AL: "Chemicals with weak skin sensitizing properties can be identified using low-density microarrays on immature dendritic cells", TOXICOLOGY LETTERS, ELSEVIER BIOMEDICAL PRESS, AMSTERDAM, NL, vol. 174, no. 1-3, 22 October 2007 (2007-10-22), pages 98-109, XP022308765, ISSN: 0378-4274, DOI: 10.1016/J.TOXLET.2007.08.015**
• **LAMBRECHTS N ET AL: "THP-1 monocytes but not macrophages as a potential alternative for CD34<+> dendritic cells to identify chemical skin sensitizers", TOXICOLOGY AND APPLIED PHARMACOLOGY, ACADEMIC PRESS, US, vol. 236, no. 2, 15 April 2009 (2009-04-15), pages 221-230, XP026029805, ISSN: 0041-008X, DOI: 10.1016/J.TAAP.2009.01.026 [retrieved on 2009-02-11]**

**(Cont. next page)**

- BERNARD F-X ET AL: "Comparison of gene expression profiles in human keratinocyte mono-layer cultures, reconstituted epidermis and normal human skin;transcriptional effects of retinoid treatments in reconstituted human epidermis", EXPERIMENTAL DERMATOLOGY, BLACKWELL MUNSGAARD, COPENHAGEN; DK, vol. 11, no. 1, 1 February 2002 (2002-02-01), pages 59-74, XP002251235, ISSN: 0906-6705, DOI: 10.1034/J.1600-0625.2002.110107.X
- PYTHON F ET AL: "Comparative DNA microarray analysis of human monocyte derived dendritic cells and MUTZ-3 cells exposed to the moderate skin sensitizer cinnamaldehyde", TOXICOLOGY AND APPLIED PHARMACOLOGY, ACADEMIC PRESS, US, vol. 239, no. 3, 15 September 2009 (2009-09-15), pages 273-283, XP026520115, ISSN: 0041-008X, DOI: 10.1016/J.TAAP.2009.06.003 [retrieved on 2009-06-12]
- SCHOETERS ET AL: "Microarray analyses in dendritic cells reveal potential biomarkers for chemical-induced skin sensitization", MOLECULAR IMMUNOLOGY, PERGAMON, GB, vol. 44, no. 12, 17 April 2007 (2007-04-17), pages 3222-3233, XP022031629, ISSN: 0161-5890, DOI: 10.1016/J.MOLIMM.2007.01.031
- HENRIK JOHANSSON ET AL: "A genomic biomarker signature can predict skin sensitizers using a cell-based in vitro alternative to animal tests", BMC GENOMICS, vol. 12, no. 1, 1 January 2011 (2011-01-01), page 399, XP55018936, ISSN: 1471-2164, DOI: 10.1186/1471-2164-12-399

**Description**

**Field of the Invention**

The present invention relates to an *in vitro* method for identifying agents capable of inducing sensitization of mammalian skin wherein the agents are capable of inducing and triggering a Type IV delayed-type hypersensitivity reaction in a mammal and arrays and analytical kits for use in such methods.

**Background**

Allergic contact dermatitis is an inflammatory skin disease that affects a significant proportion of the population. It is commonly caused by immunological responses towards chemical haptens leading to substantial economic burden for society. Current tests for sensitizing chemicals rely on animal experimentation. New legislations on the registration and use of chemicals within, e.g. the pharmaceutical and cosmetic industries, have stimulated significant research efforts to develop alternative human cell-based assays for the prediction of sensitization. The aim is to replace animal experiments with *in vitro* tests displaying a higher predictive power.

Allergic contact dermatitis (ACD) is a common inflammatory skin disease characterized by eczema and recurrent episodes of itching [1]. The disease affects a significant proportion of the population, with prevalence rates of 7.2% to 18.6% in Europe [2, 3], and the incidence is increasing due to repeated exposure to sensitizing chemicals. ACD is a type IV delayed-type hypersensitivity response caused mainly by reactive T helper 1 (Th1) and interferon (IFN)$\gamma$ producing CD8$^+$ T cells at site of contact with small chemical haptens in previously exposed, and immunologically sensitized, individuals [4]. Dendritic cells (DC) in the epidermis initiate the immune reactions by responding to haptens bound to self-molecules and activating T cell-mediated immunity.

The REACH (Registration, Evaluation, and Authorisation of Chemicals) regulation requires that all new and existing chemicals within the European Union, involving approximately 30 000 chemicals, should be tested for hazardous effects [5]. As the identification of potential sensitizers currently requires animal testing, the REACH legislation will have a huge impact on the number of animals needed for testing. Further, the 7th Amendment to the Cosmetics Directive (76/768/EEC) posed a ban on animal tests for the majority of cosmetic ingredients for human use, to be in effect by 2009, with the exceptions of some tests by 2013. Thus, development of reliable *in vitro* alternatives to experimental animals for the assessment of sensitizing capacity of chemicals is urgent. To date, no non-animal replacements are available for identification of skin sensitizing chemicals, instead the preferred assay is the mouse Local Lymph Node Assay (LLNA) [6], followed by the Guinea pig maximization test (GPMT) [7]. An *in vitro* alternative to these animal models would preferably exhibit improved reliability, accuracy and importantly correlate to human reactivity.

Dendritic cells (DCs) play key roles in the immune response by bridging the essential connections between innate and adaptive immunity. They can, upon triggering, rapidly produce large amounts of mediators, which influence migration and activation of other cells at the site of inflammation, and selectively respond to various pathogens and environmental factors, by fine-tuning the cellular response through antigen-presentation. Thus, exploring and utilizing the immunological decision-making by DCs during stimulation with sensitizers, could serve as a potent test strategy for prediction of sensitization.

WO 2010/031799 refers to a method for identifying irritating or allergenic potential of a product comprising the steps of: a) contacting the product with immature dendritic cells, b) determining the amount of 65 gene products, expressed in said cells upon contact with the product, c) subtracting the amount of gene products of these genes expressed in said cells without contact with the product from the respective amounts determined under b) to obtain a set of product-caused amounts of gene products for each of said genes, d) comparing the product-caused amounts of gene products for each of said genes obtained under c) with a test set of product-caused amounts of gene products for each of said genes, wherein said test set of product-caused amounts of gene products for each of said genes was provided from at least two allergenic products and at least two irritating products and, optionally, from at least two products which are neither allergenic nor irritating; and wherein the comparing is performed by a pattern recognition method which recognises an allergenic pattern and an irritating pattern and, optionally, a non-allergenic and non-irritating pattern; and e) identifying an allergenic potential of a product when the comparison under d) results in the recognition of an allergenic pattern and identifying an irritating potential of a product when the comparison under d) results in the recognition of an irritating pattern and, optionally, identifying a non-allergenic and non-irritating product when the comparison under d) results in the recognition of a non-allergenic and non-irritating pattern.

WO 2009/148669 refers to methods for predicting the in vivo skin sensitizing activity of chemical compounds using a combination of mammalian cell models with multiple endpoint analysis, time and concentration response curves. The methods reportedly allow the determination of a predicted in vivo sensitization value of a compound - for example, a EC3 LLNA value, a GPMT value or a IVTI value - without the use of animals.

In Szameit S et al. 2009 (Clinical & Experimental Allergy, 39: 856-868), monocyte-derived iDCs were exposed to contact sensitizers and irritants in concentrations resulting in 10-20% cytotoxicity, as determined by dose-response curves, changes in gene expression were analysed using the immune toxicity chip and a commercially available whole-genome microarray.

In Cluzel-Tailhardat M et al. 2007 (Toxicology Letters,174: 98-108), using a low-density cDNA-array, the authors analyzed the expression of 165 genes related to dendritic cell biology in human iDC following a 24h incubation with four haptens and one irritant.

Lambrechts N et al. 2009 (Toxicology and Applied Pharmacology, 236: 221-230), reports an in vitro classification model for prediction of chemical-induced skin sensitization based on gene expression signatures in human CD34+ progenitor-derived dendritic cells.

As reported in Ernard F-X et al. 2002 (Experimental Dermatology, 11: 59-74), in order to validate a model for predictive screening of dermatological drugs, the authors reportedly used a customized cDNA macro-array system containing 475 skin-related genes to analyze the gene expression patterns in human keratinocytes from different origins: (1) normal human epidermal keratinocyte mono-layer cultures, (2) the commercially available SkinEthic reconstituted human epidermis model, and (3) biopsies of normal human epidermis. In addition, the authors investigated the effects of all-trans retinoic acid, 9-cis retinoic acid, all-trans retinol and a commercialized tretinoin-containing cream (Retacnyl) on the gene expression profiles of reconstituted human epidermis.

Python F. 2009 (Toxicology and Applied Pharmacology, 239: 273-283) reports a study aimed at the identification of new dendritic cell activation markers in order to further improve the in vitro evaluation of the sensitizing potential of chemicals. It was reported that PIR, TRIM16 and two Nrf2-regulated genes, CES1 and NQO1, are modulated by most sensitizers.

Schoeters E et al. 2007 (Molecular Immunology, 44: 3222-3233) reports the use of CD34(+) progenitor-derived dendritic cells from cord blood as an in vitro alternative for Langerhans cells. The cells were reportedly exposed to four contact allergens and two irritants. Using microarray analyses the authors revealed a set of 25 genes with an altered gene expression pattern after exposure to allergens and not to irritants.

In Johansson H et al. 2011 (BMC GENOMICS. 12: 399), the authors developed a cell-based assay for the prediction of sensitizing chemicals. By analyzing the transcriptome of the human cell line MUTZ-3 after 24 h stimulation, using 20 different sensitizing chemicals, 20 non-sensitizing chemicals and vehicle controls, the authors identified a biomarker signature of 200 genes with potent discriminatory ability.

However, multifaceted phenotypes and specialized functions of different DC subpopulations, as well as their wide and scarce distribution, are complicating factors, which impede the employment of primary DCs as a test platform. Hence, there is a real need to establish accurate and reliable in vitro assays that also circumvent the problems associated with variability of and difficulty in obtaining DCs.

**Disclosure**

Thus, the development of assays based on the predictability of DC function should preferably rely on alternative cell types or mimics of in vivo DCs. For this purpose, a cell line with DC characteristics would be advantageous, as it constitutes a stable, reproducible and unlimited supply of cells. In terms of DC mimics, differentiated myelomonocytic MUTZ-3 cells are by far the prefered candidate [8]. MUTZ-3 is as an unlimited source of CD34+ DC progenitors and it can acquire, upon cytokine stimulation, phenotypes similar to immature DCs or Langerhans-like DCs [9], present antigens through CD1 d, MHC class I and II and induce specific T-cell proliferation [8]. MUTZ-3 also displays a mature transcriptional and phenotypic profile upon stimulation with inflammatory mediators [10].

The present inventors have developed a novel test principle for prediction of skin sensitizers. It has surprisingly been found that skin sensitizers can be accurately identified/predicted using DC progenitor cells, such as MUTZ-3 cells, without further differentiation in a process whereby the cells are stimulated with a panel of sensitizing chemicals, non-sensitizing chemicals, and/or other controls (e.g. vehicle controls comprising diluent only, such as DMSO and/or distilled water). This was found to substantially simplify and improve the reproducibility of the procedure.

The transcriptional response to chemical stimulation was assessed with genome-wide profiling. From data analysis, a biomarker signature of 200 transcripts was identified which completely separated the transcriptional response induced by sensitizing chemicals vs. non-sensitizing chemicals and vehicle controls. Further, the potent predictive power of the signature was illustrated, using SVM and ROC curve analysis. The biomarker signature include transcripts involved in relevant biological pathways, such as DC maturation and cytokine responses, which may shed light on the molecular interactions involved in the process of sensitization. In conclusion, a biomarker signature with potent predictive power, which represents a compelling readout for an in vitro assay useful for the identification of human sensitizing chemicals has been identified.

Hence, a first aspect of the present invention provides an in vitro method for identifying agents capable of inducing sensitization of mammalian skin wherein the agents are capable of inducing and triggering a Type IV delayed-type hypersensitivity reaction in a mammal comprising or consisting of the steps of:

a) exposing a population of dendritic cells or a population of dendritic-like cells to a test agent; and

b) measuring in the cells the expression of one or more biomarker(s) selected from the group defined in Table 3A,

wherein the expression in the cells of the two or more biomarkers measured in step (b) is indicative of the sensitizing effect of the test agent.

Preferably, the Type IV delayed-type hypersensitivity reaction is DC-mediated.

Also disclosed herein, the agent is capable of inducing and triggering a Type IV delayed-type hypersensitivity reaction at a site of epidermal contact in a mammal.

The mammal may be any domestic or farm animal. Preferably, the mammal is a rat, mouse, guinea pig, cat, dog, horse or a primate. Most preferably, the mammal is human. As discussed above, *in vivo* methods of determining sensitisation are known in the art. A preferred method is the Local lymph node assay (for details, see Basketter, D.A., et al., Local lymph node assay - validation, conduct and use in practice. Food Chem Toxicol, 2002. 40(5): p. 593-8). A further suitable, but less preferred, method is the guinea pig maximization test (for details, see Magnusson, B. and A.M. Kligman, The identification of contact allergens by animal assay. The guinea pig maximization test. J Invest Dermatol, 1969. 52(3): p. 268-76).

By "dendritic-like cells" we mean non-dendritic cells that exhibit functional and phenotypic characteristics specific to dendritic cells such as morphological characteristics, expression of costimulatory molecules and MHC class II molecules, and the ability to pinocytose macromolecules and to activate resting T cells.

Also disclosed herein, the dendritic-like cells are CD34$^+$ dendritic cell progenitors. Optionally, the CD34$^+$ dendritic cell progenitors can acquire, upon cytokine stimulation, the phenotypes of presenting antigens through CD1 d, MHC class I and II, induce specific T-cell proliferation, and/or displaying a mature transcriptional and phenotypic profile upon stimulation with inflammatory mediators (i.e. similar phenotypes to immature dendritic cells or Langerhans-like dendritic cells).

Dendritic cells may be recognized by function, by phenotype and/or by gene expression pattern, particularly by cell surface phenotype. These cells are characterized by their distinctive morphology, high levels of surface MHC-class II expression and ability to present antigen to CD4+ and/or CD8+ T cells, particularly to naïve T cells (Steinman et al. (1991) Ann. Rev. Immunol. 9: 271).

The cell surface of dendritic cells is unusual, with characteristic veil-like projections, and is characterized by expression of the cell surface markers CD11c and MHC class II. Most DCs are negative for markers of other leukocyte lineages, including T cells, B cells, monocytes/macrophages, and granulocytes. Subpopulations of dendritic cells may also express additional markers including 33D1, CCR1, CCR2, CCR4, CCR5, CCR6, CCR7, CD1 a-d, CD4, CD5, CD8alpha, CD9, CD11b, CD24, CD40, CD48, CD54, CD58, CD80, CD83, CD86, CD91, CD117, CD123 (IL3Ra), CD134, CD137, CD150, CD153, CD162, CXCR1, CXCR2, CXCR4, DCIR, DC-LAMP, DC-SIGN, DEC205, E-cadherin, Langerin, Mannose receptor, MARCO, TLR2, TLR3 TLR4, TLR5, TLR6, TLR9, and several lectins.

The patterns of expression of these cell surface markers may vary along with the maturity of the dendritic cells, their tissue of origin, and/or their species of origin. Immature dendritic cells express low levels of MHC class II, but are capable of endocytosing antigenic proteins and processing them for presentation in a complex with MHC class II molecules. Activated dendritic cells express high levels of MHC class 11, ICAM-1 and CD86, and are capable of stimulating the proliferation of naive allogeneic T cells, e. g. in a mixed leukocyte reaction (MLR).

Functionally, dendritic cells or dendritic-like cells may be identified by any convenient assay for determination of antigen presentation. Such assays may include testing the ability to stimulate antigen- primed and/or naive T cells by presentation of a test antigen, followed by determination of T cell proliferation, release of IL-2, and the like.

By "expression" we mean the level or amount of a gene product such as mRNA or protein.

Methods of detecting and/or measuring the concentration of protein and/or nucleic acid are well known to those skilled in the art, see for example Sambrook and Russell, 2001, Cold Spring Harbor Laboratory Press.

Preferred methods for detection and/or measurement of protein include Western blot, North-Western blot, immunosorbent assays (ELISA), antibody microarray, tissue microarray (TMA), immunoprecipitation, *in situ* hybridisation and other immunohistochemistry techniques, radioimmunoassay (RIA), immunoradiometric assays (IRMA) and immunoenzymatic assays (IEMA), including sandwich assays using monoclonal and/or polyclonal antibodies. Exemplary sandwich assays are described by David et al., in US Patent Nos. 4,376,110 and 4,486,530. Antibody staining of cells on slides may be used in methods well known in cytology laboratory diagnostic tests, as well known to those skilled in the art.

Typically, ELISA involves the use of enzymes which give a coloured reaction product, usually in solid phase assays. Enzymes such as horseradish peroxidase and phosphatase have been widely employed. A way of amplifying the phosphatase reaction is to use NADP as a substrate to generate NAD which now acts as a coenzyme for a second enzyme system. Pyrophosphatase from *Escherichia coli* provides a good conjugate because the enzyme is not present in tissues, is stable and gives a good reaction colour. Chemi-luminescent systems based on enzymes such as luciferase can also be used.

Conjugation with the vitamin biotin is frequently used since this can readily be detected by its reaction with enzyme-linked avidin or streptavidin to which it binds with great specificity and affinity.

Preferred methods for detection and/or measurement of nucleic acid (e.g. mRNA) include southern blot, northern blot, polymerase chain reaction (PCR), reverse transcriptase PCR (RT-PCR), quantitative real-time PCR (qRT-PCR), nanoarray, microarray, macroarray, autoradiography and *in situ* hybridisation.

In one embodiment the method comprises exposing a separate population of the dendritic cells or dendritic-like cells to a negative control agent that does not sensitize human skin, and measuring in the cells the expression of the one or more biomarker(s) measured in step (b). Hence, a sensitizing effect of the test agent is indicated in the event that the expression in the cell population of the one or more biomarker(s) measured in step (b) is/are different from the expression in the negative control sample.

Also disclosed herein, the negative control agent is a solvent for use with the test or control agents. Hence, the negative control may be DMSO and/or distilled water.

Also disclosed herein, the expression of one or more biomarkers measured in step (b) of the dendritic cells or dendritic-like cells prior to test agent exposure is used as a negative control.

A further aspect of the disclosure comprises exposing a separate population of the dendritic cells or dendritic-like cells to a positive control agent that sensitizes human skin and measuring in the cells the expression of the one or more biomarker(s) measured in step (b). Hence, a sensitizing effect of the test agent is indicated in the event that the expression in the cell population of the one or more biomarker(s) measured in step (b) is/are similar to or the same as the expression in the positive control sample.

Preferably the method comprises, in step (b), measuring the expression of at least one biomarker selected from the group consisting of:

> i) taste receptor, type 2, member 5 (TAS2R5),
> ii) keratinocyte growth factor-like protein 1/2/hypothetical protein FLJ20444 (KGFLP1/2/ FLJ20444),
> iii) transmembrane anterior posterior transformation 1 (TAPT1),
> iv) sprouty homolog 2 (SPRY2),
> v) fatty acid synthase (FASN),
> vi) B-cell CLL/lymphoma 7A (BCL7A),
> vii) solute carrier family 25, member 32 (SLC25A32),
> viii) ferritin, heavy polypeptide pseudogene 1 (FTHP1),
> ix) ATPase, H+ transporting, lysosomal 50/57kDa, V1 subunit H (ATP6V1H),
> x) squalene epoxidase (SQLE), and
> xi) histone cluster 1, H1e (HIST1H1E).

Hence, in one embodiment the expression of taste receptor, type 2, member 5 (TAS2R5) is measured in step (b). In a further embodiment, in step (b), the expression of keratinocyte growth factor-like protein 1/2/hypothetical protein FLJ20444 (KGFLP1/2/ FLJ20444) is measured. The method may comprise measuring the expression of transmembrane anterior posterior transformation 1 (TAPT1) in step (b). In one embodiment, the method comprises measuring the expression of sprouty homolog 2 (SPRY2) in step (b). However, a further embodiment the method, in step (b), comprises measuring the expression of fatty acid synthase (FASN). The method may comprise measuring the expression of B-cell CLL/lymphoma 7A (BCL7A) in step (b). It may also comprise measuring the expression of solute carrier family 25, member 32 (SLC25A32) in step (b). It may additionally comprise, in step (b), measuring the expression of ferritin, heavy polypeptide pseudogene 1 (FTHP1). A still further embodiment comprises measuring the expression of ATPase, H+ transporting, lysosomal 50/57kDa, V1 subunit H (ATP6V1 H) in step (b). In another embodiment step (b) comprises measuring the expression of squalene epoxidase (SQLE). In yet another embodiment the expression of histone cluster 1, H1e (HIST1H1E) is measured in step (b).

The method may comprise or consist of measuring, in step (b), the expression of at least 2 biomarkers from Table 3A, for example, at least 3, 4, 5, 6, 7, 8, 9, 10 or 11 biomarkers from Table 3A. Also disclosed herein, the method comprises or consists of measuring the expression of fatty acid synthase (FASN) and squalene epoxidase (SQLE) in step (b).

The method may additionally or alternatively comprise or consist of, measuring in step (b) the expression of at least 2 biomarkers from Table 3B, for example, at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123,124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, or at least 189 biomarkers from Table 3B.

Thus, the expression of all of the biomarkers in Table 3A and/or all of the biomarkers in Table 3B may be measured in step (b).

In a preferred embodiment, step (b) comprises or consists of measuring the expression of a nucleic acid molecule encoding the two or more biomarker(s). The nucleic acid molecule may be a cDNA molecule or an mRNA molecule. Preferably, the nucleic acid molecule is an mRNA molecule.

Also disclosed herein, the expression of the one or more biomarker(s) in step (b) is performed using a method selected from the group consisting of Southern hybridisation, Northern hybridisation, polymerase chain reaction (PCR), reverse transcriptase PCR (RT-PCR), quantitative real-time PCR (qRT-PCR), nanoarray, microarray, macroarray, autoradiography and *in situ* hybridisation. Preferably, the expression of the one or more biomarker(s) is measured using a DNA microarray.

The method disclosed herein may comprise measuring the expression of the two or more biomarker(s) in step (b) using two or more binding moieties, each capable of binding selectively to a nucleic acid molecule encoding one of the biomarkers identified in Table 3A. Also disclosed herein, the one or more binding moieties each comprise or consist of a nucleic acid molecule. Also disclosed herein, the one or more binding moieties each comprise or consist of DNA, RNA, PNA, LNA, GNA, TNA or PMO. Preferably, the one or more binding moieties each comprise or consist of DNA. Also disclosed herein, the one or more binding moieties are 5 to 100 nucleotides in length. Alternatively, they are 15 to 35 nucleotides in length.

Suitable binding agents (also referred to as binding molecules) may be selected or screened from a library based on their ability to bind a given nucleic acid, protein or amino acid motif, as discussed below.

Also disclosed herein, the binding moiety comprises a detectable moiety.

By a "detectable moiety" we include a moiety which permits its presence and/or relative amount and/or location (for example, the location on an array) to be determined, either directly or indirectly.

Suitable detectable moieties are well known in the art.

For example, the detectable moiety may be a fluorescent and/or luminescent and/or chemiluminescent moiety which, when exposed to specific conditions, may be detected. Such a fluorescent moiety may need to be exposed to radiation (*i.e.* light) at a specific wavelength and intensity to cause excitation of the fluorescent moiety, thereby enabling it to emit detectable fluorescence at a specific wavelength that may be detected.

Alternatively, the detectable moiety may be an enzyme which is capable of converting a (preferably undetectable) substrate into a detectable product that can be visualised and/or detected. Examples of suitable enzymes are discussed in more detail below in relation to, for example, ELISA assays.

Hence, the detectable moiety may be selected from the group consisting of: a fluorescent moiety; a luminescent moiety; a chemiluminescent moiety; a radioactive moiety (for example, a radioactive atom); or an enzymatic moiety. Preferably, the detectable moiety comprises or consists of a radioactive atom. The radioactive atom may be selected from the group consisting of technetium-99m, iodine-123, iodine-125, iodine-131, indium-111, fluorine-19, carbon-13, nitrogen-15, oxygen-17, phosphorus-32, sulphur-35, deuterium, tritium, rhenium-186, rhenium-188 and yttrium-90.

Clearly, the agent to be detected (such as, for example, the one or more biomarkers in the test sample and/or control sample described herein and/or an antibody molecule for use in detecting a selected protein) must have sufficient of the appropriate atomic isotopes in order for the detectable moiety to be readily detectable.

Alternatively, the detectable moiety of the binding moiety is a fluorescent moiety.

The radio- or other labels may be incorporated into the biomarkers present in the samples disclosed herein and/or the binding moieties in known ways. For example, if the binding agent is a polypeptide it may be biosynthesised or may be synthesised by chemical amino acid synthesis using suitable amino acid precursors involving, for example, fluorine-19 in place of hydrogen. Labels such as $^{99m}$Tc, $^{123}$I, $^{186}$Rh, $^{188}$Rh and $^{111}$In can, for example, be attached *via* cysteine residues in the binding moiety. Yttrium-90 can be attached via a lysine residue. The IODOGEN method (Fraker et al (1978) Biochem. Biophys. Res. Comm. 80, 49-57) can be used to incorporate $^{123}$I. Reference ("Monoclonal Antibodies in Immunoscintigraphy", J-F Chatal, CRC Press, 1989) describes other methods in detail. Methods for conjugating other detectable moieties (such as enzymatic, fluorescent, luminescent, chemiluminescent or radioactive moieties) to proteins are well known in the art.

It will be appreciated by persons skilled in the art that biomarkers in the sample(s) to be tested may be labelled with a moiety which indirectly assists with determining the presence, amount and/or location of said proteins. Thus, the moiety may constitute one component of a multicomponent detectable moiety. For example, the biomarkers in the sample(s) to be tested may be labelled with biotin, which allows their subsequent detection using streptavidin fused or otherwise joined to a detectable label.

In another embodiment of first aspect of the present invention step (b) comprises determining the expression of the protein of the two or more biomarker(s). The method may comprise measuring the expression of the two or more biomarker(s) in step (b) using two or more binding moieties each capable of binding selectively to one of the biomarkers identified in Table 3A. Also disclosed herein, the one or more binding moieties may comprise or consist of an antibody or an antigen-binding fragment thereof such as a monoclonal antibody or fragment thereof.

The term "antibody" includes any synthetic antibodies, recombinant antibodies or antibody hybrids, such as but not limited to, a single-chain antibody molecule produced by phage-display of immunoglobulin light and/or heavy chain variable and/or constant regions, or other immunointeractive molecules capable of binding to an antigen in an immunoassay format that is known to those skilled in the art.

We also include the use of antibody-like binding agents, such as affibodies and aptamers.

A general review of the techniques involved in the synthesis of antibody fragments which retain their specific binding sites is to be found in Winter & Milstein (1991) Nature 349, 293-299.

Additionally, or alternatively, one or more of the first binding molecules may be an aptamer (see Collett et al., 2005, Methods 37:4-15).

Molecular libraries such as antibody libraries (Clackson et al, 1991, Nature 352, 624-628; Marks et al, 1991, J Mol Biol 222(3): 581-97), peptide libraries (Smith, 1985, Science 228(4705): 1315-7), expressed cDNA libraries (Santi et al (2000) J Mol Biol 296(2): 497-508), libraries on other scaffolds than the antibody framework such as affibodies (Gunneriusson et al, 1999, Appl Environ Microbiol 65(9): 4134-40) or libraries based on aptamers (Kenan et al, 1999, Methods Mol Biol 118, 217-31) may be used as a source from which binding molecules that are specific for a given motif are selected for use in the methods disclosed.

The molecular libraries may be expressed *in vivo* in prokaryotic cells (Clackson *et al,* 1991, *op. cit.;* Marks *et al,* 1991, *op. cit.)* or eukaryotic cells (Kieke et al, 1999, Proc Natl Acad Sci USA, 96(10):5651-6) or may be expressed *in vitro* without involvement of cells (Hanes & Pluckthun, 1997, Proc Natl Acad Sci USA 94(10):4937-42; He & Taussig, 1997, Nucleic Acids Res 25(24):5132-4; Nemoto et al, 1997, FEBS Lett, 414(2):405-8).

In cases when protein based libraries are used, the genes encoding the libraries of potential binding molecules are often packaged in viruses and the potential binding molecule displayed at the surface of the virus (Clackson *et al,* 1991, *supra;* Marks *et al*, 1991, *supra;* Smith, 1985, *supra).*

Perhaps the most commonly used display system is filamentous bacteriophage displaying antibody fragments at their surfaces, the antibody fragments being expressed as a fusion to the minor coat protein of the bacteriophage (Clackson *et al,* 1991, *supra;* Marks *et al,* 1991, *supra).* However, other suitable systems for display include using other viruses (EP 39578), bacteria (Gunneriusson *et al,* 1999, *supra;* Daugherty et al, 1998, Protein Eng 11(9):825-32; Daugherty et al, 1999, Protein Eng 12(7):613-21), and yeast (Shusta et al, 1999, J Mol Biol 292(5):949-56).

In addition, display systems have been developed utilising linkage of the polypeptide product to its encoding mRNA in so-called ribosome display systems (Hanes & Pluckthun, 1997, *supra;* He & Taussig, 1997, *supra;* Nemoto *et al*, 1997, *supra),* or alternatively linkage of the polypeptide product to the encoding DNA (see US Patent No. 5,856,090 and WO 98/37186).

The variable heavy ($V_H$) and variable light ($V_L$) domains of the antibody are involved in antigen recognition, a fact first recognised by early protease digestion experiments. Further confirmation was found by "humanisation" of rodent antibodies. Variable domains of rodent origin may be fused to constant domains of human origin such that the resultant antibody retains the antigenic specificity of the rodent parented antibody (Morrison et al (1984) Proc. Natl. Acad. Sci. USA 81, 6851-6855).

That antigenic specificity is conferred by variable domains and is independent of the constant domains is known from experiments involving the bacterial expression of antibody fragments, all containing one or more variable domains. These molecules include Fab-like molecules (Better et al (1988) Science 240, 1041); Fv molecules (Skerra et al (1988) Science 240, 1038); single-chain Fv (ScFv) molecules where the $V_H$ and $V_L$ partner domains are linked via a flexible oligopeptide (Bird et al (1988) Science 242, 423; Huston et al (1988) Proc. Natl. Acad. Sci. USA 85, 5879) and single domain antibodies (dAbs) comprising isolated V domains (Ward et al (1989) Nature 341, 544). A general review of the techniques involved in the synthesis of antibody fragments which retain their specific binding sites is to be found in Winter & Milstein (1991) Nature 349, 293-299.

The antibody or antigen-binding fragment may be selected from the group consisting of intact antibodies, Fv fragments *(e.g.* single chain Fv and disulphide-bonded Fv), Fab-like fragments (e.g. Fab fragments, Fab' fragments and F(ab)$_2$ fragments), single variable domains (e.g. $V_H$ and $V_L$ domains) and domain antibodies (dAbs, including single and dual formats [*i.e.* dAb-linker-dAb]). Preferably, the antibody or antigen-binding fragment is a single chain Fv (scFv).

The one or more binding moieties may alternatively comprise or consist of an antibody-like binding agent, for example an affibody or aptamer.

By "scFv molecules" we mean molecules wherein the $V_H$ and $V_L$ partner domains are linked via a flexible oligopeptide.

The advantages of using antibody fragments, rather than whole antibodies, are several-fold. The smaller size of the fragments may lead to improved pharmacological properties, such as better penetration of solid tissue. Effector functions of whole antibodies, such as complement binding, are removed. Fab, Fv, ScFv and dAb antibody fragments can all be expressed in and secreted from *E. coli,* thus allowing the facile production of large amounts of the said fragments.

Whole antibodies, and F(ab')$_2$ fragments are "bivalent". By "bivalent" we mean that the said antibodies and

F(ab')$_2$ fragments have two antigen combining sites. In contrast, Fab, Fv, ScFv and dAb fragments are monovalent, having only one antigen combining sites.

The antibodies may be monoclonal or polyclonal. Suitable monoclonal antibodies may be prepared by known techniques, for example those disclosed in "Monoclonal Antibodies: A manual of techniques", H Zola (CRC Press, 1988) and in "Monoclonal Hybridoma Antibodies: Techniques and applications", J G R Hurrell (CRC Press, 1982).

When potential binding molecules are selected from libraries, one or more selector peptides having defined motifs are usually employed. Amino acid residues that provide structure, decreasing flexibility in the peptide or charged, polar or hydrophobic side chains allowing interaction with the binding molecule may be used in the design of motifs for selector peptides. For example:

(i) Proline may stabilise a peptide structure as its side chain is bound both to the alpha carbon as well as the nitrogen;
(ii) Phenylalanine, tyrosine and tryptophan have aromatic side chains and are highly hydrophobic, whereas leucine and isoleucine have aliphatic side chains and are also hydrophobic;
(iii) Lysine, arginine and histidine have basic side chains and will be positively charged at neutral pH, whereas aspartate and glutamate have acidic side chains and will be negatively charged at neutral pH;
(iv) Asparagine and glutamine are neutral at neutral pH but contain a amide group which may participate in hydrogen bonds;
(v) Serine, threonine and tyrosine side chains contain hydroxyl groups, which may participate in hydrogen bonds.

Typically, selection of binding molecules may involve the use of array technologies and systems to analyse binding to spots corresponding to types of binding molecules.

The one or more protein-binding moieties may comprise a detectable moiety. The detectable moiety may be selected from the group consisting of a fluorescent moiety, a luminescent moiety, a chemiluminescent moiety, a radioactive moiety and an enzymatic moiety.

Also disclosed herein, step (b) may be performed using an assay comprising a second binding agent capable of binding to the one or more proteins, the second binding agent also comprising a detectable moiety. Suitable second binding agents are described in detail above in relation to the first binding agents.

Thus, the proteins of interest in the sample to be tested may first be isolated and/or immobilised using the first binding agent, after which the presence and/or relative amount of said biomarkers may be determined using a second binding agent.

Also disclosed herein, the second binding agent is an antibody or antigen-binding fragment thereof; typically a recombinant antibody or fragment thereof. Conveniently, the antibody or fragment thereof is selected from the group consisting of: scFv; Fab; a binding domain of an immunoglobulin molecule. Suitable antibodies and fragments, and methods for making the same, are described in detail above.

Alternatively, the second binding agent may be an antibody-like binding agent, such as an affibody or aptamer.

Alternatively, where the detectable moiety on the protein in the sample to be tested comprises or consists of a member of a specific binding pair (e.g. biotin), the second binding agent may comprise or consist of the complimentary member of the specific binding pair (e.g. streptavidin).

Where a detection assay is used, it is preferred that the detectable moiety is selected from the group consisting of: a fluorescent moiety; a luminescent moiety; a chemiluminescent moiety; a radioactive moiety; an enzymatic moiety. Examples of suitable detectable moieties for use in the methods of the present disclosure are described above.

Preferred assays for detecting serum or plasma proteins include enzyme linked immunosorbent assays (ELISA), radioimmunoassay (RIA), immunoradiometric assays (IRMA) and immunoenzymatic assays (IEMA), including sandwich assays using monoclonal and/or polyclonal antibodies. Exemplary sandwich assays are described by David et al in US Patent Nos. 4,376,110 and 4,486,530. Antibody staining of cells on slides may be used in methods well known in cytology laboratory diagnostic tests, as well known to those skilled in the art.

Also disclosed herein, the assay is an ELISA (Enzyme Linked Immunosorbent Assay) which typically involves the use of enzymes which give a coloured reaction product, usually in solid phase assays. Enzymes such as horseradish peroxidase and phosphatase have been widely employed. A way of amplifying the phosphatase reaction is to use NADP as a substrate to generate NAD which now acts as a coenzyme for a second enzyme system. Pyrophosphatase from *Escherichia coli* provides a good conjugate because the enzyme is not present in tissues, is stable and gives a good reaction colour. Chemiluminescent systems based on enzymes such as luciferase can also be used.

Conjugation with the vitamin biotin is frequently used since this can readily be detected by its reaction with enzyme-linked avidin or streptavidin to which it binds with great specificity and affinity.

Also disclosed herein, the assay used for protein detection is conveniently a fluorometric assay. Thus, the detectable moiety of the second binding agent may be a fluorescent moiety, such as an *Alexa* fluorophore (for example Alexa-647).

Preferably, step (b) is performed using an array. The array may be a bead-based array or a surface-based array. The array may be selected from the group consisting of: macroarray; microarray; nanoarray.

Also disclosed herein, the method is for identifying agents capable of inducing a hypersensitivity response in human skin. Preferably, the hypersensitivity response is a cell-mediated hypersensitivity response, for example, a type IV hypersensitivity response. Preferably, the method is for identifying agents capable of inducing allergic contact dermatitis (ACD) (i.e. the hypersensitivity response is ACD).

Also disclosed herein, the population of dendritic cells or population of dendritic-like cells is a population of dendritic cells. Preferably, the dendritic cells are primary dendritic cells. Preferably, the dendritic cells are myeloid dendritic cells.

The population of dendritic cells or dendritic-like cells is preferably mammalian in origin. Preferably, the mammal is a rat, mouse, guinea pig, cat, dog, horse or a primate. Most preferably, the mammal is human.

Also disclosed herein, the population of dendritic cells or population of dendritic-like cells is a population of dendritic-like cells, preferably myeloid dendritic-like cells.

Also disclosed herein, the dendritic-like cells express at least one of the markers selected from the group consisting of CD54, CD86, CD80, HLA-DR, CD14, CD34 and CD1a, for example, 2, 3, 4, 5, 6 or 7 of the markers. Also disclosed herein, the dendritic-like cells express the markers CD54, CD86, CD80, HLA-DR, CD14, CD34 and CD1a.

Also disclosed herein, the dendritic-like cells may be derived from myeloid dendritic cells. Preferably the dendritic-like cells are myeloid leukaemia-derived cells. Preferably, the myeloid leukaemia-derived cells are selected from the group consisting of KG-1, THP-1, U-937, HL-60, Monomac-6, AML-193 and MUTZ-3. Most preferably, dendritic-like cells are MUTZ-3 cells. MUTZ-3 cells are human acute myelomonocytic leukemia cells that were deposited with Deutsche Sammlung für Mikroorganismen und Zellkulturen GmbH (DSMZ), (Inhoffenstraße 7B, Braunschweig, Germany) on 15 May 1995 (www.dsmz.de; deposit no. ACC 295).

Also disclosed herein, the dendritic-like cells, after stimulation with cytokine, present antigens through CD1d, MHC class I and II and/or induce specific T-cell proliferation.

Also disclosed herein, the negative control agent(s) is/are selected from the group consisting of 1-Butanol, 4-Aminobenzoic acid, Benzaldehyde, Chlorobenzene, Diethyl phthalate, Dimethyl formamide, Ethyl vanillin, Glycerol, Isopropanol, Lactic acid, Methyl salicylate, Octanoic acid, Propylene glycol, Phenol, p-ydroxybenzoic acid, Potassium permanganate, Salicylic acid, Sodium dodecyl sulphate, Tween 80 and Zinc sulphate.

Also disclosed herein, the method may comprise the use of at least 2 negative control agents (i.e. non-sensitizing agents), for example, at least 3, 4, 5, 6, 7, 8, 9, 10, 11, 12, 13, 14, 15, 16, 17, 18, 19 or at least 20 negative control agents.

Also disclosed herein, the positive control agent(s) is/are selected from the group consisting of 2,4-Dinitrochlorobenzene, Oxazolone, Potassium dichromate, Kathon CH (MC/MCI), Formaldehyde, 2-Aminophenol, 2-nitro-1,4-Phenylendiamine, p-Phenylendiamine, Hexylcinnamic aldehyde, 2-Hydroxyethyl acrylate, 2-Mercaptobenzothiazole, Glyoxal, Cinnamaldehyde, Isoeugenol, Ethylendiamine, Resorcinol, Cinnamic alcohol, Eugenol, Penicillin G or Geraniol.

Also disclosed herein, the method may comprise the use of at least 2 positive control (i.e. sensitizing agents) are provided, for example, at least 3, 4, 5, 6, 7, 8, 9, 10, 11, 12, 13, 14, 15, 16, 17, 18, 19 or at least 20 positive control agents.

Also disclosed herein, the method is indicative of whether the test agent is or is not a sensitizing agent. Also disclosed herein, the method is indicative of the sensitizing potency of the sample to be tested.

Also disclosed herein, the method is indicative of the local lymph node assay (LLNA) classification of the sensitizing potency of the sample to be tested. For a detailed description of LLNA see Basketter, D.A., et al., Local lymph node assay - validation, conduct and use in practice. Food Chem Toxicol, 2002. 40(5): p. 593-8.

Also disclosed herein, the method is indicative of the guinea pig maximization test classification of the sensitizing potency of the sample to be tested. For a detailed description of the guinea pig maximization test see Magnusson, B. and A.M. Kligman, The identification of contact allergens by animal assay. The guinea pig maximization test. J Invest Dermatol, 1969. 52(3): p. 268-76.

Also disclosed herein, the method is indicative that the test agent is either, a non-sensitizer, a weak sensitizer, a moderate sensitizer, a strong sensitizer or an extreme sensitizer. The decision value and distance in PCA correlates with sensitizer potency.

Generally, skin sensitizing agents are determined with an ROC AUC of at least 0.55, for example with an ROC AUC of at least, 0.60, 0.65, 0.70, 0.75, 0.80, 0.85, 0.90, 0.95, 0.96, 0.97, 0.98, 0.99 or with an ROC AUC of 1.00. Preferably, skin sensitizing agents are determined with an ROC AUC of at least 0.85, and most preferably with an ROC AUC of 1.

Typically, agents capable of inducing sensitization are identified using a support vector machine (SVM), such as those available from http://cran.r-project.org/web/packages/e1071/index.html (e.g. e1071 1.5-24). However, any other suitable means may also be used.

Support vector machines (SVMs) are a set of related supervised learning methods used for classification and regression. Given a set of training examples, each marked as belonging to one of two categories, an SVM training algorithm builds a model that predicts whether a new example falls into one category or the other. Intuitively, an SVM model is a representation of the examples as points in space, mapped so that the examples of the separate categories are divided by a clear gap that is as wide as possible. New examples are then mapped into that same space and predicted to belong to a category based on which side of the gap they fall on.

More formally, a support vector machine constructs a hyperplane or set of hyperplanes in a high or infinite dimensional space, which can be used for classification, regression or other tasks. Intuitively, a good separation is achieved by the hyperplane that has the largest distance to the nearest training datapoints of any class (so-called functional margin), since in general the larger the margin the lower the generalization error of the classifier. For more information on SVMs, see for example, Burges, 1998, Data Mining and Knowledge Discovery, 2:121-167.

Also disclosed herein, the SVM is 'trained' prior to performing the methods of the invention using biomarker profiles of known agents (namely, known sensitizing or non-sensitizing agents). By running such training samples, the SVM is able to learn what biomarker profiles are associated with agents capable of inducing sensitization. Once the training process is complete, the SVM is then able whether or not the biomarker sample tested is from a sensitizing agent or a non-sensitizing agent.

This allows test agents to be classified as sensitizing or non-sensitizing. Moreover, by training the SVM with sensitizing agents of known potency (i.e. non-sensitizing, weak, moderate, strong or extreme sensitizing agents), the potency of test agents can also be identified comparatively.

However, this training procedure can be by-passed by pre-programming the SVM with the necessary training parameters. For example, agents capable of inducing sensitization be identified according to the known SVM parameters using the SVM algorithm detailed in Table 5, based on the measurement of all the biomarkers listed in Table 3(A) and 1(B).

It will be appreciated by skilled persons that suitable SVM parameters can be determined for any combination of the biomarkers listed Table 3 by training an SVM machine with the appropriate selection of data (i.e. biomarker measurements from cells exposed to known sensitizing and/or non-sensitizing agents).

Preferably, the method disclosed herein has an accuracy of at least 73%, for example 74%, 75%, 76%, 77%, 78%, 79%, 80%, 81%, 82%, 83%, 84%, 85%, 86%, 87%, 88%, 89%, 90%, 91 %, 92%, 93%, 94%, 95%, 96%, 97%, 98%, 99% or 100% accuracy.

Preferably, the method disclosed herein has a sensitivity of at least 73%, for example 74%, 75%, 76%, 77%, 78%, 79%, 80%, 81%, 82%, 83%, 84%, 85%, 86%, 87%, 88%, 89%, 90%, 91 %, 92%, 93%, 94%, 95%, 96%, 97%, 98%, 99% or 100% sensitivity.

Preferably, the method disclosed herein has a specificity of at least 68%, for example 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91 %, 92%, 93%, 94%, 95%, 96%, 97%, 98%, 99% or 100% specificity.

By "accuracy" we mean the proportion of correct outcomes of a method, by "sensitivity" we mean the proportion of all positive chemicals that are correctly classified as positives, and by "specificity" we mean the proportion of all negative chemicals that are correctly classified as negatives.

A second aspect of the invention provides an array for use in the method of the first aspect of the invention, wherein the binding moieties of the array consist of binding moieties for between 10 and 194 of the biomarkers defined in Table 3, and wherein the binding moieties of the array comprise two or more binding moieties as defined in the first aspect. Also disclosed herein, the array comprises one or more binding moieties as defined above. Also disclosed herein, the binding moieties are (collectively) capable of binding to all of the biomarkers defined in Table 3A. Also disclosed herein, the binding moieties are (collectively) capable of binding to all of the biomarkers defined in Table 3B. Preferably, the binding moieties are (collectively) capable of binding to all of the biomarkers defined in Table 3A and Table 3B.

The binding moieties may be immobilised.

Arrays per se are well known in the art. Typically they are formed of a linear or two-dimensional structure having spaced apart (i.e. discrete) regions ("spots"), each having a finite area, formed on the surface of a solid support. An array can also be a bead structure where each bead can be identified by a molecular code or colour code or identified in a continuous flow. Analysis can also be performed sequentially where the sample is passed over a series of spots each adsorbing the class of molecules from the solution. The solid support is typically glass or a polymer, the most commonly used polymers being cellulose, polyacrylamide, nylon, polystyrene, polyvinyl chloride or polypropylene. The solid supports may be in the form of tubes, beads, discs, silicon chips, microplates, polyvinylidene difluoride (PVDF) membrane, nitrocellulose membrane, nylon membrane, other porous membrane, non-porous membrane (e.g. plastic, polymer, perspex, silicon, amongst others), a plurality of polymeric pins, or a plurality of microtitre wells, or any other surface suitable for immobilising proteins, polynucleotides and other suitable molecules and/or conducting an immunoassay. The binding processes are well known in the art and generally consist of cross-linking covalently binding or physically adsorbing a protein molecule, polynucleotide or the like to the solid support. Alternatively, affinity coupling of the probes via affinity-tags or similar constructs may be employed. By using well-known techniques, such as contact or non-contact printing, masking or photolithography, the location of each spot can be defined. For reviews see Jenkins, R.E., Pennington, S.R. (2001, Proteomics, 2,13-29) and Lal et al (2002, Drug Discov Today 15;7(18 Suppl):S143-9).

Typically the array is a microarray. By "microarray" we include the meaning of an array of regions having a density of discrete regions of at least about 100/cm$^2$, and preferably at least about 1000/cm$^2$. The regions in a microarray have typical dimensions, *e.g.* diameter, in the range of between about 10-250 $\mu$m, and are separated from other regions in the array by about the same distance. The array may alternatively be a macroarray or a nanoarray.

Once suitable binding molecules (discussed above) have been identified and isolated, the skilled person can manufacture an array using methods well known in the art of molecular biology; see Examples below.

A third aspect of the present invention provides the use of two or more biomarkers selected from the group defined in Table 3A in combination for identifying hypersensitivity response sensitising agents. Preferably, all of the biomarkers defined in Table 3A and Table 3B are used collectively for identifying hypersensitivity response sensitising agents. Preferably, the use is consistent with the method described in the first aspect of the invention.

A fourth aspect of the invention provides an analytical kit for use in a method according the first aspect of the invention, comprising or consisting of:

A) an array according to the second aspect of the invention; and

B) instructions for performing the method according to the first aspect of the invention.

The analytical kit may comprise one or more control agents. Preferably, the analytical kit comprises or consists of the above features, together with one or more negative control agents and/or one or more positive control agents.

The scope of the presently claimed invention is defined by the claims.

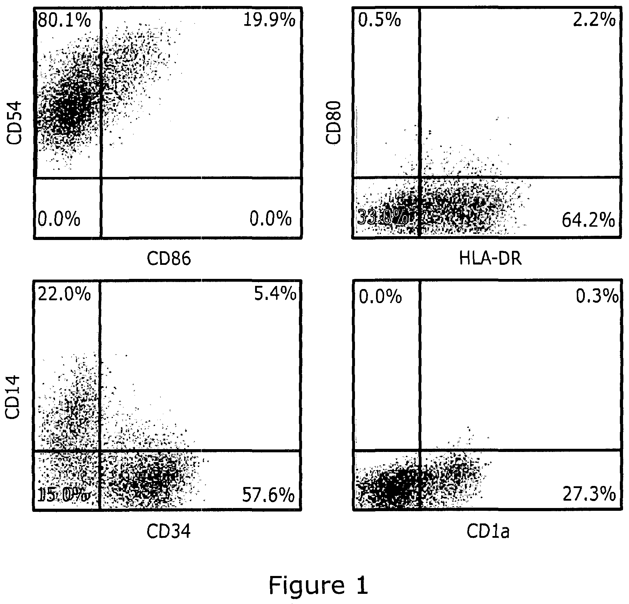

**Figure 1. Phenotype of MUTZ-3 cells prior to stimulation with sensitizing and non-sensitizing chemicals**
Cell surface expression levels of CD14, CD1 a, CD34, CD54, CD80, CD86 and HLA-DR were assessed with flow cytometry. Gates were set to exclude debris and dead cells, and quadrants were established by comparing with relevant isotype controls. Results are shown from one representative experiment out of six.

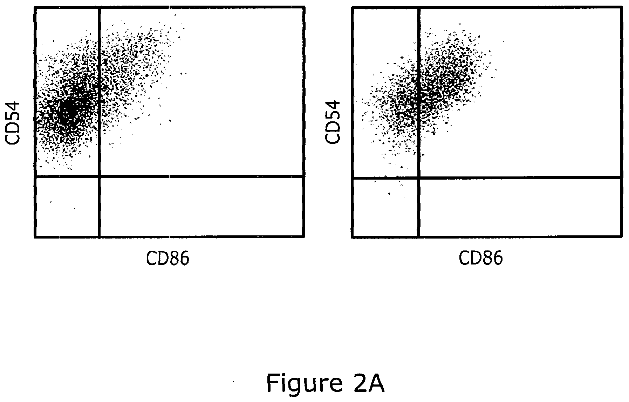

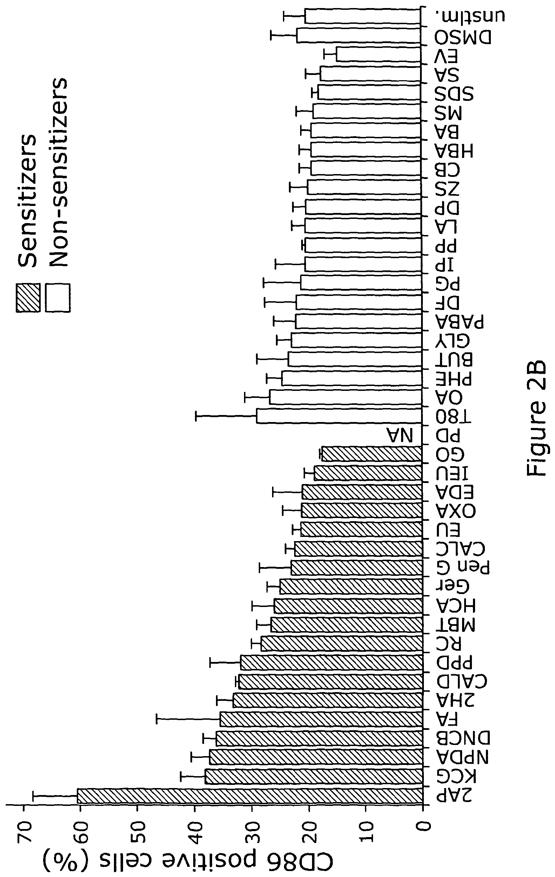

**Figure 2. Changes in CD86 expression following stimulation with sensitizing and non-sensitizing chemicals**
Cell surface expression levels of CD86 were monitored after stimulation with chemicals for 24 h. A). Chemical-induced upregulation of CD86, in terms of changes in frequency of positive cells, were determined by flow cytometry, as exemplified by the comparison of 2-aminophenol-stimulated cells (right dotplot) and unstimulated controls (left dotplot). Results are shown from one representative experiment out of three. Gates were set to exclude debris and dead cells, and quadrants were established by comparing with relevant isotype controls. B) Compilation of frequencies of CD86-positive cells after 24 h of stimulation. Statistical analysis was performed using paired Student's *t* test. *$p < 0.05$, # $p < 0.01$. NA, not analysed.

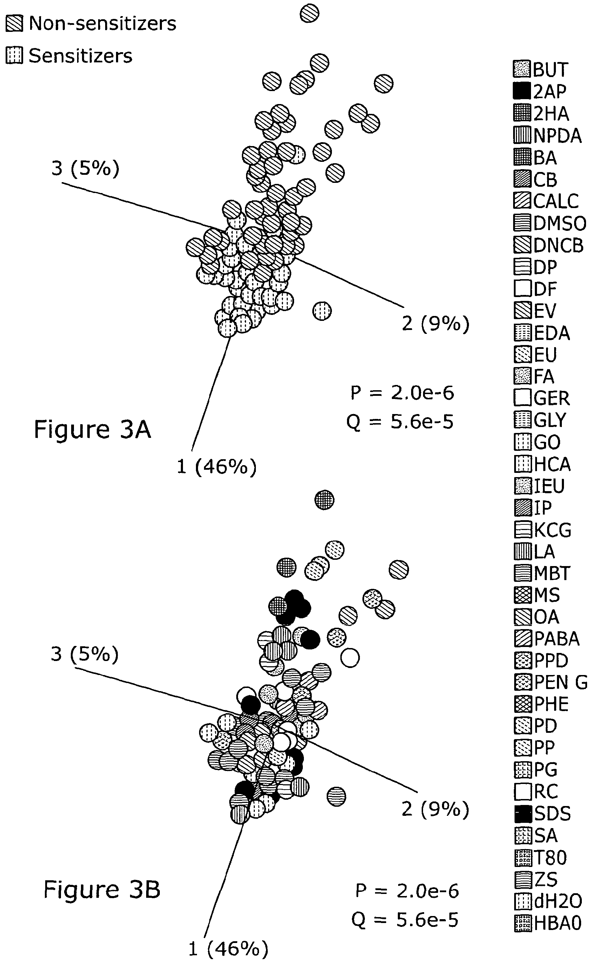

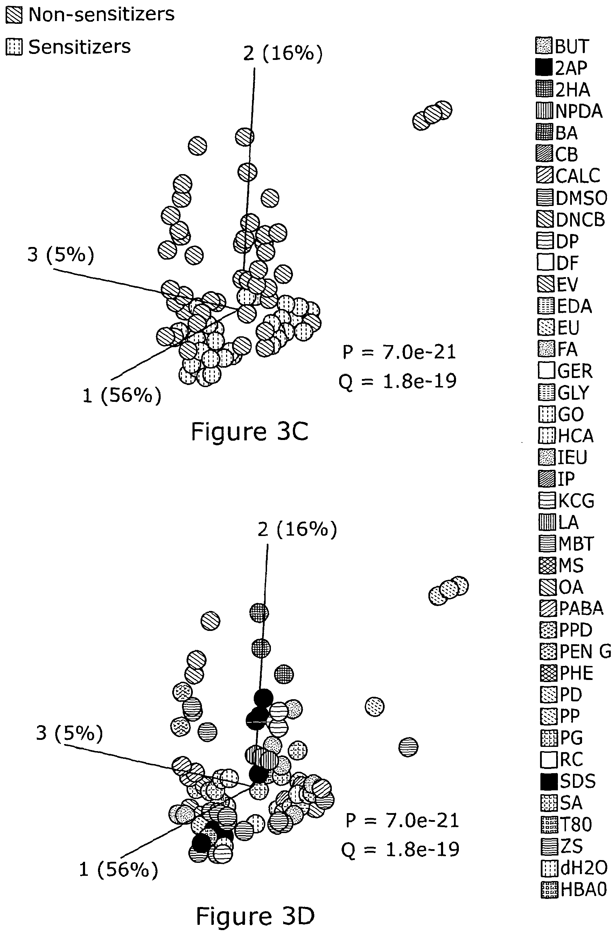

**Figure 3. Principal component analysis of transcripts differentially expressed after chemical stimulation**
mRNA levels in MUTZ-3 cells stimulated for 24 h with 20 sensitizing and 20 non-sensitizing chemicals were assessed with transcritomics using Affymetrix Human Gene 1.0 ST arrays. Structures and similarities in the gene expression dataset were investigated using principal component analysis (PCA) in the software Qlucore. A) PCA of genes differentially expressed in cells stimulated with sensitizing (red) as compared to non-sensitizing (green) chemicals (1010 genes identified with one-way ANOVA). B) PCA of genes differentially expressed in cells stimulated with sensitizing as compared to non-sensitizing chemicals (1010 genes), but now samples are coloured by the compound used for stimulation. C) PCA of genes differentially expressed when comparing the different stimulations with 2-way ANOVA (1137 genes). Samples are coloured according to sensitizing (red) and non-sensitizing (green) chemicals. D) PCA of genes differentially expressed when comparing the different stimulations with 2-way ANOVA (1137 genes), but now samples are coloured by the compound used for stimulation. P, p-value from ANOVA. Q, p-value corrected for multiple hypothesis testing.

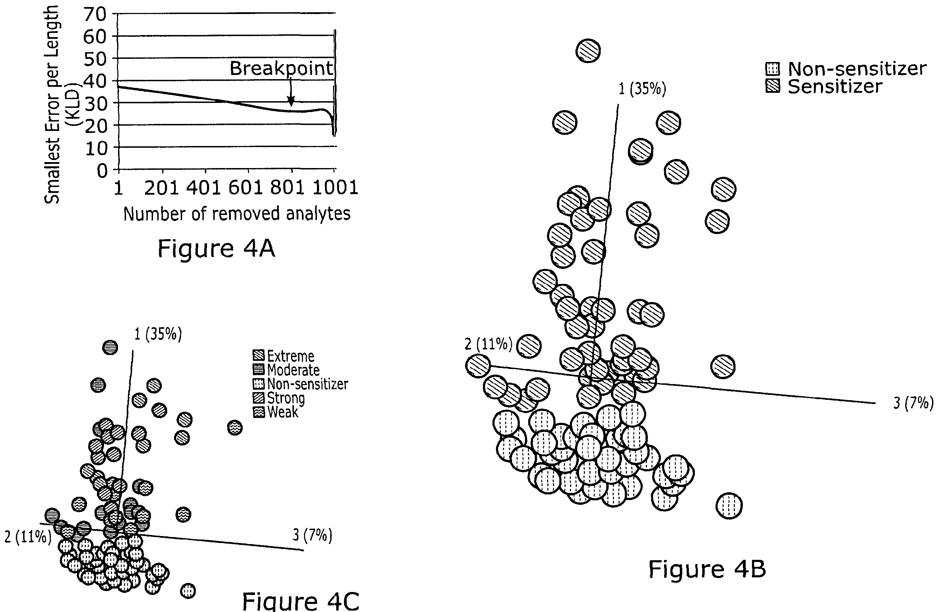

**Figure 4. Identification and PCA analysis of "Prediction Signature"** A) The number of differentially expressed significant genes in cells stimulated with sensitizing as compared to non-sensitizing chemicals (1010 genes) was reduced using Backward Elimination. The lowest KLD is observed after elimination of 810 analytes, referred to as the Breakpoint. The remaining 200 genes is considered to be the top predictors in the data set, and is termed "Prediction Signature". B) Complete separation between sensitizers (red) and non-sensitizers (green) is observed with PCA of the "Prediction Signature". C) Same PCA as in B, now with samples coloured according to their potency in LLNA.

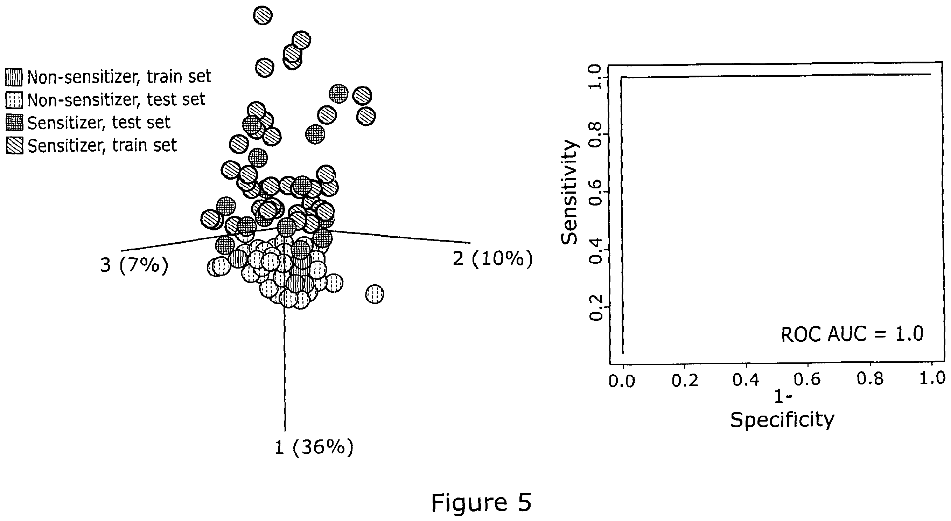

**Figure 5. Validation of selection procedure of "Prediction Signature"**
The method by which the "Prediction Signature" was constructed was validated by repeating the process on 70% of the data set, selected at random. The remaining 30% of data was used as a test set for signature validation. A) PCA demonstrates that the "Test Gene Signature" can separate sensitizers from non-sensitizers. Only the samples of the 70% training set, displayed in bright colours, were used to build the space of the first three principal components. The test set samples, displayed in dark colours, were plotted into this space based on expression levels of the analytes in the "Test Gene Signature". B) An SVM was trained on the 70% training set, and validated with the 30% test set. The area under the ROC curve of 1.0 proves that the group belonging of all samples in the test set was correctly predicted, demonstrating the strength of the "Test Gene Signature", and by association also the strength of our "Prediction Signature".

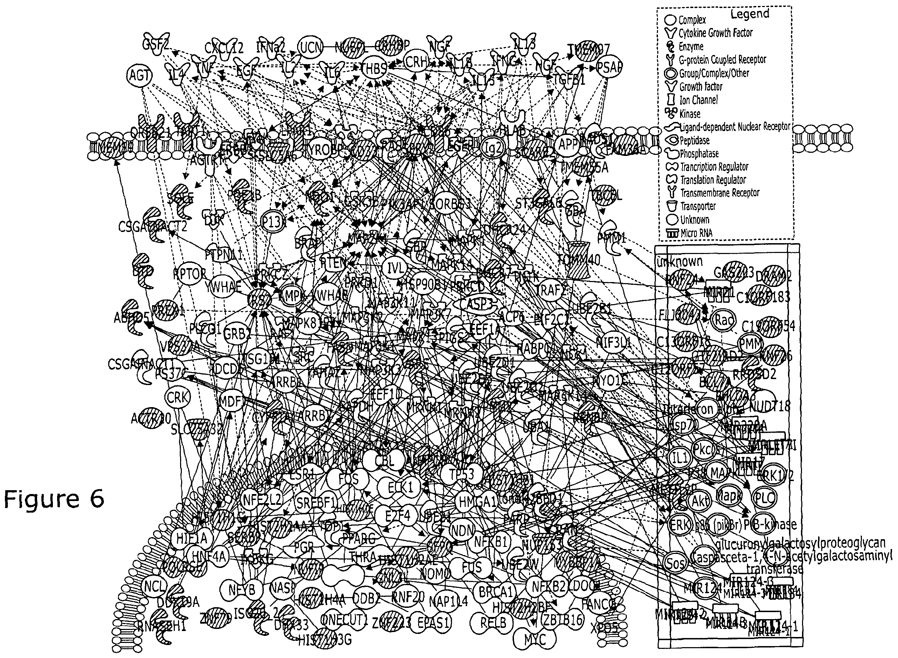

**Figure 6. The interactome of the "Prediction Signature"**
Interactome of the 200 molecules (orange) and molecules connecting theses according to evidence from IPA. Direct interactions are shown as solid lines and indirect as dotted lines.

**EXAMPLES**

Allergic contact dermatitis is an inflammatory skin disease that affects a significant proportion of the population. This is commonly caused by immunological responses towards chemical haptens leading to substantial economic burden for society. Current test of sensitizing chemicals rely on animal experimentation. New legislations on the registration and use of chemicals within e.g. pharmaceutical and cosmetic industries have stimulated significant research efforts to develop alternative human cell-based assays for the prediction of sensitization. The aim is to replace animal experiments with *in vitro* tests displaying a higher predictive power.

We have developed a novel cell-based assay for the prediction of sensitizing chemicals. By analyzing the transcriptome of the human cell line MUTZ-3 after 24 h stimulation with 20 different sensitizing chemicals, 20 non-sensitizing chemicals and vehicle controls, we have identified a biomarker signature of 200 genes with potent discriminatory ability. Using a Support Vector Machine for supervised classification, the prediction performance of the assay revealed an accuracy of 100%, sensitivity of 100% and specificity of 100%. In addition, categorizing the chemicals according to the LLNA assay, this gene signature could also predict potency. The identified markers are involved in biological pathways with immunological relevant functions, which can shed light on the process of human sensitization.

A gene signature predicting sensitization using a human cell line *in vitro* has been developed. This easy and robust cell-based assay can completely replace or drastically reduce current test systems, using experimental animals. Being based on human biology, the assay is considered to be more relevant and more accurate for predicting sensitization in humans than the traditional animal-based tests.

**Results**

**The cellular rational of the in vitro cell culture system**

Acting as the link between innate and adaptive immunity, DCs are essential immunoregulatory cells of the immune system. Their unique property to recognize antigen for the purpose of initiating T cell responses, and their potent regulatory function in skewing immune responses, makes them targets for assay development. However, primary DCs constitute a heterogeneous and minor population of cells not suited for screening. The obvious advantages of using a cell line with characteristics compared to primary DCs for the basis of a predictive test are stability, reproducibility and unlimited supply of cells. So far, no leukemia with obvious DC-like properties has been reported, probably due to the fact that the characteristics of this cell type are determined by a complex terminal differentiation process that can occur only post-cell division [11], and thus, the generation of DC-like cell lines relies on available myeloid leukemia cell lines. MUTZ-3 is a human acute myelomonocytic leukemia cell line with a potent ability to differentiate into DCs, present antigens and induce specific T-cell proliferation. Among the available myeloid human cell lines, MUTZ-3 is by far the preferred candidate. Similar to immature primary DCs, MUTZ-3 progenitor express CD1a, HLA-DR and CD54, as well as low levels of CD80 and CD86 (Figure 1). The MUTZ-3 population also contains three subpopulations of CD14$^+$, CD34$^+$ and double negative cells, previously reported to be transitional differentiation steps from a proliferative CD34$^+$ progenitor into a non-proliferative CD14$^+$ DC precursor [9]. Consequently, we utilized constitutively differentiating progenitor MUTZ-3 cells as the basis for the test system.

**CD86 surface expression in response to sensitizer stimulation:** CD86 is the most extensively studied biomarker for sensitization to date, in cell-systems such as monocyte derived dendritic cells (MoDCs) or dendritic cell-like human cell lines and their progenitors, such as THP-1, U-937 and KG-1. Thus, as a reference, cell surface expression of CD86 was measured with flow cytometry after 24h stimulation with 20 sensitizers and 20 non-sensitizers, as well as with vehicle controls (Table 1). CD86 was significantly up-regulated on cells stimulated with 2-Aminophenol, Kathon CG, 2-nitro-1,4-Phenylendiamine, 2,4-Dinitrochlorobenzene, 2-Hydroxyethyl acrylate, Cinnamic aldehyde, p-Phenylendiamine, Resorcinol, and 2-Mercaptobenzothiazole. Hence, an assay based on measurement of a single biomarker, such as CD86, would give a sensitivity of 47% and a specificity of 100%. Consequently, CD86 cannot classify skin sensitizers, using a cell based system such as MUTZ-3.

**Analysis of the transcriptional profiles in chemically stimulated MUTZ-3 cells:**

The genomic expression arrays were used to test 20 sensitizers and 20 non-sensitizers, in triplicates, and vehicle controls such as DMSO and distilled water, the latter in twelve replicates. In total, a data set was generated based on 144 samples. RMA normalization and quality controls of the samples revealed that the Oxazolone and Cinnamic aldehyde samples were significant outliers and had to be removed, or they would have dominated the data set prohibiting biomarker identification (data not shown). In addition, one of the replicates of potassium permanganate had to be removed due to a faulty array. This left a data set consisting of 137 samples, each with data from measurements of 33,297 transcripts. In order to mine the data set for information specific for sensitizers vs. non-sensitizers, the software Qlucore Omics Explorer 2.1 was used, which enable real time principal component analysis (PCA) analysis, while sorting the input genes after desired criteria, e.g. sensitizers and non-sensitizers, based on ANOVA p-value selection. Figure 3A and 3B show PCAs based on 1010 transcripts with a p-value of $\leq 2.0 \times 10^{-6}$ from ANOVA analysis, comparing sensitizing vs. non-sensitizing chemicals. As can be seen in figure 3A, a clear distinction can be made between the two groups, with non-sensitizers forming a condensed cloud in the lower part of the figure (green), while sensitizers stretch upwards in various directions (red). However, a complete separation is not achieved between the two groups at this level of significance. From figure 3B, now coloured according to stimulation, it is evident that one or more replicate of Glyoxal, Eugenol, Hexylcinnamic aldehyde, Isoeugenol, Resorcinol, Penicillin G and Ethylendiamine group together with the control group. In addition, one replicate or more of the non-sensitizers Tween 80, Octanoic acid and Phenol tend to group with the sensitizers. Figure 3C and 3D show PCA plots based on 1137 genes that all have a p-value of $\leq 7.0 \times 10^{-21}$, when comparing the different stimulations. Identifying this large number of genes at this level of significance provides strong indications of the power of the data set. In figure 3D, it is clear that the replicates group together, indicating high quality data. The triplicate samples of Potassium dichromate have a discrete profile, which demonstrate a substantial impact of the cells compared to non-sensitizers. Furthermore, 2-Hydroxyethyl acrylate, 2-Aminophenol, Kathon CG, Formaldehyde, 2-nitro-1,4-Phenylendiamine, 2,4-Dinitrochlorobenzoic acid, p-Phenylendiamine, 2-Mercaptobenzothiazole, Cinnamic alcohol and Resorcinol have replicates that group together, separate from the negative group. Still, as can be seen in figure 3C as well as in 3A, complete separation is not achieved with neither of the gene signatures of 1010 and 1137 genes respectively.

**Backward elimination identifies genes with the most discriminatory power:**

Even though the data set contains genes with p-values down to $1 \times 10^{-17}$, lowering the p-value cutoff did not achieve complete separation between sensitizers and non-sensitizers. Gene signatures entirely selected on p-values does not provide the best possible predictive power, since a low p-value is no guarantee that a gene provides any additional information. To further reduce the number of transcripts for a predictive biomarker signature, we employed an algorithm for backward elimination (Figure 4A). The algorithm removes genes one by one while taking into account not only the impact of genes individually, but how they perform collectively with the entire selected gene signature. For each gene eliminated, the Kullback-Leibler divergence (KLD) value is lowered, until a breakpoint is reached, at which point 200 genes remained. Continuing eliminating genes at this point causes the KLD to rise again, indicating that information is being lost (Figure 4A). Therefore, the 200 genes with lowest KLD value were selected for further analysis. PCA of the 200 analytes revealed that they have the ability to completely separate sensitizers vs. non-sensitizers, indicating that these transcripts can be used as predictors for sensitizing properties of unknown samples (Figure 4B). Importantly, by coloring the samples in the PCA by their potency, according to LLNA, it is clear that potency can be predicted by these genes, as well (Figure 4C). The 200 genes are termed the "Prediction Signature" and their identity is listed in Table 3.

**Validation of the analysis method used to identify the Prediction Signature:** To validate the predictive power of our signature, we used a supervised learning method called the Support Vector Machine (SVM) [12], which maps the data from a training set in space in order to maximize the separation of gene expression induced by sensitizing and non-sensitizing chemicals. As training set, 70% of the data set was selected randomly and the entire selection process (as described above) was repeated. Starting with 29,141 transcripts, the signature was reduced to 200 transcripts, termed "Test Gene Signature", using ANOVA filtering and backward elimination. The remaining 30% of the data set was used to test the signature obtained. The partitioning of the data set into subsets of 70% training data set and 30% test data set was done in a stratified random manner, meaning that the proportion of sensitizers and non-sensitizers in the complete data set are maintained in both the subsets, although the samples included in either of the two subsets are selected at random. Thereafter, the "Test Gene Signature" was used to train an SVM model with the training set, and the predictive power of the model was assessed with the test set. Figure 5A shows a PCA plot based on the "Test Gene Signature" and the samples of the test set. Clearly, the separation between sensitizers (red) and non-sensitizers (green) resembles the one observed for the "Prediction Signature" in Figure 4B. In the PCA of Figure 5A, the samples of the sensitizing and non-sensitizing chemicals of the test set have been colored dark red and dark green respectively, indicating that they are not contributing to the principal components of the plot, but are merely plotted based on their expression values of the selected "Test Gene Signature". As can be seen, sensitizers from the test set group with sensitizers from the training set, while non-sensitizers from the test set group with non-sensitizers from the training set. This is a very intuitive way of predicting the group belonging of the samples in the test set, using only the pattern recognition of the eye. The outcome of the SVM training and validation can be seen in Figure 5B, where an area under the ROC curve of 1 confirms the ability of the "Test Gene Signature" to predict which group the sample belonged to in the test set. The prediction performance of the assay reveals an accuracy of 96%, sensitivity of 100% and specificity of 92%. While this experiment does not validate the prediction power of the "Prediction Signature" per se, it does indeed validate the method by which it has been selected, supporting the claim that the "Prediction Signature" is capable of accurately predict sensitizing properties of unknown samples.

**Molecular functions and canonical pathways of the "Prediction Signature".**

Using Ingenuity Pathways Analysis (IPA, Ingenuity Systems Inc.), 184 of the 200 molecules in the signature were characterized with regard to functions and known (canonical) pathways. The remaining 16 molecules could not be mapped to any IPA entries. The dominating functions identified were small molecule biochemistry (38 molecules), cell death (33), lipid metabolism (24), hematological system development (19), cellular growth and proliferation (16), molecular transport (15), cell cycle (15) and carbohydrate metabolism (15), see table 4 for details. 67 of the 184 molecules were involved in the listed functions. Of the remaining 117 molecules, 30 were known from a variety of human diseases and molecular functions, such as described biomarkers (SCARB2, RFC2, VPS37A and BCL7A) and drug targets (ABAT). Most of these molecules are metabolic markers. In the signature as a whole, there are several drug targets, such as HMGCR, HMOX1, ABAT, RXRA, CD33, MAP2K1, MAPK13 and CD86. Two are described for skin disorders: CD86 (psoriasis) and RXRA (eczema). The signature also contains skin development (DHCR24) and dendritic cell markers (MAP2K1, NLRP12 and RFC2).

Pathways possibly invoked by the molecules in the signature were also investigated using IPA. Those most highly populated were NRF2 mediated oxidative response (10), xenobiotic metabolism signaling (8), LPS/IL-1 mediated inhibition of RXR function (6), aryl hydrocarbon receptor signaling (6) and protein kinase A signaling (6). The five highest ranked of these pathways are all known to take part in reactions provoked by foreign substances, xenobiotics. Xenobiotics are natural or synthetic chemical compounds, foreign to the human body.

**Conclusions**

Allergic contact dermatitis (ACD) is an inflammatory skin disease caused by dysregulated adaptive immune responses to allergens [13]. Small molecular weight chemicals, so-called haptens, can bind self-proteins in the skin, which enables internalisation of the protein-bound allergenic chemical by skin dendritic cell (DC). DCs, under the influence of the local microenvironment, process the protein-hapten complex, migrate to the local lymph nodes and activate naïve T cells. The initiation and development of allergen-specific responses, mainly effector CD8+ T cells and Th1 cells, and production of immunoregulatory proteins, are hallmarks of the immune activation observed in ACD. This T-cell mediated type IV hypersensitivity reaction is characterised by symptoms such as rash, blisters and itching. ACD is the most common manifestation of immunotoxicity observed in humans [13] and hundreds of chemicals have been shown to cause sensitization in skin [14]. The driving factors and molecular mechanisms involved in sensitization are still unknown even though intense research efforts have been carried out to identify the immunological responses towards allergenic chemicals. The REACH legislation requires that all chemicals produced over 1 ton/year are tested for hazardous properties such as toxicity and allergenicity [5], which increase the demand for accurate assays for predictive power. Today, the identification of potential human sensitizers relies on animal experimentation, in particular the murine local lymph node assay (LLNA) [6]. The LLNA is based upon measurements of proliferation induced in draining lymph nodes of mice after chemical exposure [15]. Chemicals are defined as sensitizers if they provoke a three-fold increase in proliferation compared to control, and the amount of chemical required for the increase is the EC3 value. Thus, the LLNA can also be used to categorize the chemicals based on sensitisation potency. However, LLNA is in many ways not optimal. Besides the obvious ethical reasons, the assay is also time consuming and expensive. Human sensitization data often stem from human maximization tests (HMT) [16] and human patch tests (HPT). In an extensive report from the Interagency Coordinating Committee on the Validation of Alternative Methods (ICCVAM), the performance characteristics of LLNA were compared to other available animal-based methods and human sensitization data (HMT and HPT) [17]. The LLNA performance in comparison to human data (74 assessments) revealed an *accuracy* of 72%, a *sensitivity* of 72% and a *specificity* of 67%. *Accuracy* is defined as the proportion of correct outcomes of a method, *sensitivity* is the proportion of all positive chemicals that are correctly classified as positives, and *specificity* is the proportion of all negative chemicals that are correctly classified as negatives. Thus, there is a clear need to develop more reliable test methods for sensitization. Additionally, the 7th Amendment to the Cosmetics Directive (76/768/EEC) poses a complete ban on using animal experimentation for testing cosmetic ingredients by 2013 when a scientific reliable method is available. Thus, there is a great need from the industry for reliable predictive test methods that are based on human cells. Various human cell lines and primary cells involved in sensitization have been evaluated as predictive test system, such as epithelial cells, dendritic cells and T cells, however no validated test assay is currently available. Various single biomarkers have been suggested to be upregulated upon stimulation with sensitizing chemicals, such as CD40, CD80, CD54, CXCL8, IL-1 β, MIP-1β, p38 MAPK as reviewed in [18], yet singlehanded, none of them have the predictive power to discriminate between sensitizing and non-sensitizing chemicals. CD86 is among the markers most extensively studied; however, determining the expression level of this marker in our assay is relevant but not sufficient as readout for sensitization (Figure 2). Only 9 out of 19 sensitizing chemicals induced a significant upregulation of CD86. Instead, it is our firm belief that the analyses of biomarker signatures are superior to any single biomarker. We therefore utilized the power of complete-genome transcriptomics and screened the gene regulation induced by a large set of well-defined chemicals and controls. The large number of differentially expressed genes in MUTZ-3 cells stimulated with sensitizing chemicals as compared to non-sensitizing controls, as identified with ANOVA (Figure 3) revealed that MUTZ-3 indeed has a capacity to differentiate between these two groups, thus being a suitable cell system for *in vitro* assays for sensitization. Efforts have been made to create assays based on full genome analysis in various cell systems, such as CD34[+]-progenitor cells-derived DCs [19-21]. While such assays might provide *in vivo* like environments, one could argue that primary cells are not well suited for an assay of this great commercial interest. Moreover, the ethical aspect needs to be considered in such a system. Furthermore, previous efforts within *in vitro* assay development for sensitization that rely on full genome analysis have used a very limited set of testing compounds. To date, this study is the largest study performed within this area, utilizing 20 positive and 20 negative training compounds. Efforts have been made by us to divide these training compounds into two subsets, for training and testing respectively. While these experiments have resulted in successful predictions (data not shown), it is our experience that sensitizing compounds differ greatly in their induced gene expression profile, as can be seen in figure 3D. In this perspective, we wished to include as many training compounds as possible when identifying our "Prediction Signature". Thus, we did not exclude any compounds for validation. Instead, we have validated the method by which the "Prediction Signature" was identified, by subdividing the samples into training and test sets at random, using unseen data for validation, as seen in Figure 5. By including all sensitizing compounds, with a wide range of reactive mechanisms as well as sensitizing potencies, we argue that we have identified a prediction signature that is well suited for predicting sensitizing properties of unknown samples. Of important note, our "Prediction Signature" is able to predict the potency of sensitizing compounds, such as those defined by the LLNA, as is demonstrated in Figure 4C. However, the potency predicted by LLNA and that of our classifier do not match for all samples. Notably, the moderate sensitizer 2-Hydroxyethyl acrylate show strong resemblance of strong and extreme sensitizers with respect to their gene expression profile. Similarly, the moderate sensitizers Ethylendiamine, Hexylcinnamic aldehyde, and Glyoxal group together with weak sensitizers. These findings provide vast implications that sensitizing potency, as defined, may need revising. We argue that our "Prediction Signature" may be used for such classifications.

In conclusion, we present an *in vitro* assay, based on a MUTZ-3 cell system that with an identified "Prediction Signature" consisting of 200 genes, which have the ability to correctly classify a sample as sensitizer or non-sensitizer. In addition, this assay can predict the potency of sensitizing compounds, and may be used to revise such classifications.

**Materials and methods**

Chemicals

A panel of chemicals consisting of 20 sensitizers and 20 non-sensitizers were used for cell stimulations. The sensitizers were 2,4-Dinitrochlorobenzene, Cinnamaldehyde, Resorcinol, Oxazolone, Glyoxal, 2-Mercaptobenzothiazole, Eugenol, Isoeugenol, Cinnamic alcohol and *ρ*-Phenylendiamine, Formaldehyde, Ethylendiamine, 2-Hydroxyethyl acrylate, Hexylcinnamic aldehyde, Potassium Dichromate, Penicillin G, Katchon CG (MCI/MI), 2-aminophenol, Geraniol and 2-nitro-1,4-Phenylendiamine (Table 1). The non-sensitizers were Sodium dodecyl sulphate, Salicylic acid, Phenol, Glycerol, Lactic acid, Chlorobenzene, *p*-Hydrobenzoic acid, Benzaldehyde, Diethyl Phtalate and Octanoic acid, Zinc sulphate, 4-Aminobenzoic acid, Methyl salicylate, Ethyl vanillin, Isopropanol, Dimethyl formamide, 1-Butanol, Potassium permanganate, Propylene glycol and Tween 80 (Table 1). All chemicals were from Sigma-Aldrich, St. Louis, MO, USA. Compounds were dissolved in either Dimethyl sulfoxide (DMSO) or distilled water. Prior to stimulations, the cytotoxicity of all compounds were monitored, using Propidium Iodide (PI) (BD Biosciences, San Diego, CA) using protocol provided by the manufacturer. The relative viability of stimulated cells was calculated as

$$Relative\ viability = \frac{fraction\ of\ viable\ stimulated\ cells}{fraction\ of\ viable\ unstimulated\ cells} \cdot 100$$

For toxic compounds, the concentration yielding 90% relative viability (Rv90) was used. For untoxic compounds, a concentration of 500 $\mu$M was used when possible. For non-toxic compounds that were insoluble at 500 $\mu$M in medium, the highest soluble concentration was used. For compounds dissolved in DMSO, the in-well concentration was 0.1% DMSO. The vehicle and concentrations used for each compound are listed in Table 2.

Chemical exposure of the cells

The human myeloid leukaemia-derived cell line MUTZ-3 (DSMZ, Braunschweig, Germany) was maintained in $\alpha$-MEM (Thermo Scientific Hyclone, Logan, UT) supplemented with 20% (volume/volume) fetal calf serum (Invitrogen, Carlsbad, CA) and 40 ng/ml rhGM-CSF (Bayer HealthCare Pharmaceuticals, Seattle, WA), as described [10]. Prior to each experiment, the cells were immunophenotyped using flow cytometry as a quality control. Cells were seeded in 6-well plates at 200.000 cells/ml. Stock solutions of each compound was prepared in either DMSO or distilled water, and were subsequently diluted so the in-well concentrations corresponded to the Rv90 value, and in-well concentrations of DMSO were 0.1%. Cells were incubated for 24h at 37°C and 5% $CO_2$. Thereafter, cells were harvested and analysed with flow cytometry. In parallel, harvested cells were lysed in TRIzol reagent (Invitrogen) and stored at -20°C until RNA extraction. Stimulations with chemicals were performed in three individual experiments, so that triplicates samples were obtained.

Phenotypic analysis with flow cytometry

All cell surface staining and washing steps was performed in PBS containing 1% BSA (w/v). Cells were incubated with specific mouse mAbs for 15 min at 4°C. The following mAbs were used for flow cytometry: FITC-conjugated CD1a (DakoCytomation, Glostrup, Denmark), CD34, CD86, and HLA-DR (BD Biosciences), PE-conjugated CD14 (DakoCytomation), CD54 and CD80 (BD Biosciences). Mouse IgG1, conjugated to FITC or PE were used as isotype controls and PI was used to assess cell viability (BD Biosciences). FACSDiva software was used for data acquisition with FACSCanto II instrument (BD Bioscience). 10,000 events were acquired and gates were set based on light scatter properties to exclude debris and nonviable cells. Further data analysis was performed using FCS Express V3 (De Novo Software, Los Angeles, CA).

Preparation of cRNA and gene chip hybridization

RNA isolation and gene chip hybridization was performed as described [22]. Briefly, RNA from unstimulated and chemical-stimulated MUTZ-3 cells, from triplicate experiments, were extracted and analysed. The preparation of labeled sense DNA was performed according to Affymetrix GeneChip Whole Transcript (WT) Sense Target Labeling Assay (100 ng Total RNA Labeling Protocol) using the recommended kits and controls (Affymetrix, Santa Clara, CA). Hybridization, washing and scanning of the Human Gene 1.0 ST Arrays were performed according to the manufacturer's protocol (Affymetrix).

Microarray data analysis and statistical methods

The microarray data were normalised and quality checked with the RMA algorithm, using Affymetrix Expression Console (Affymetrix). Genes that were significantly regulated when comparing sensitizers with non-sensitizers were identified using one-way ANOVA, with false discovery rate (FDR) as a correction for multiple hypothesis testing. In order to reduce the large number of identified significant gene, we applied an algorithm developed in-house for Backward Elimination of analytes (Carlsson et al, unpublished). With this method, we train and test a Support Vector Machine (SVM) model [12] with leave-one out cross-validation, with one analyte left out. This process is iterated until each analyte has been left out once. For each iteration, a Kullback-Leibler divergence (KLD) is recorded, yielding $N$ KLDs, where $N$ is the number of analytes. The analyte that was left out when the smallest KLD was observed is considered to provide the least information in the data set. Thus, this analyte is eliminated and the iterations proceed, this time with $N$-1 analytes. In this manner, the analytes are eliminated one by one until a panel of markers remain that have been selected based on each analyte's ability to discriminate between sensitizers and non-sensitizers. The script for Backwards Eliminations was programmed for R [23], with the additional package e1071 [24]. ANOVA analyses and visualisation of results were performed in Qlucore Omics Explorer 2.1 (Qlucore, Lund, Sweden). The selected biomarker profile of 200 transcripts were designated the "Prediction Signature".

Validation of the method for identification of the "Prediction Signature"

In the absence of an external test data set, the data set was divided into a training set of 70% and a test set of 30% of the samples. The division was performed randomly, while maintaining the proportions of sensitizers and non-sensitizers in each subset at the same ratio as in the complete data set. A biomarker signature was identified in the training set using ANOVA filtering and Backward Elimination, as described above. This test signature was used to train an SVM, using the training set, which was thereafter applied to predict the samples of the test set. The distribution of the area under the Receiver Operating Characteristic (ROC) curve [25] was used as a measurement of the performance of the model.

Assessment of biological functions of "Prediction Signature" using pathway analysis

In order to investigate the biological functions the gene profile was analyzed using the Ingenuity Pathway Analysis software, IPA, (Ingenuity Systems, Inc. Mountain View, USA). The 200 top genes resulting from Backward Elimination were analyzed using the 'build' and 'Path Explorer' functions to build an interactome of the core genes from the "Prediction Signature" and connecting molecules suggested by IPA. The 200 molecules in the "Prediction Signature" were connected using the shortest known paths. In this process only human evidence from primary cells, cell lines and epidermal tissue was used. All molecules except for endogenous and chemical drugs were allowed in the network and all kinds of connections were allowed. Known 'Functions' and 'Canonical Pathways' from IPA were mapped to the interactome using the 'Overlay' function.

**Abbreviations**

| | |
|---|---|
| ACD, | atopic contact dermatitis; |
| AML, | acute myeloid leukemia cell; |
| APC, | Antigen Presenting Cell; |
| DC, | Dendritic Cell; |
| GM-CSF, | Granulocyte macrophage colony-stimulating factor; |
| GPMT, | Guinea pig maximization test; |
| HMT, | Human Maximation Test; |
| HPTA, | Human Patch Test Allergen; |
| IL, | Interleukin; |
| LLNA, | Local Lymph Node Assay; |
| PCA, | Principal Component Analysis |

**References**

1. Akhavan, A. and S.R. Cohen, The relationship between atopic dermatitis and contact dermatitis. Clin Dermatol, 2003. 21(2): p. 158-62.
2. Mortz, C.G., et al., Prevalence of atopic dermatitis, asthma, allergic rhinitis, and hand and contact dermatitis in adolescents. The Odense Adolescence Cohort Study on Atopic Diseases and Dermatitis. Br J Dermatol, 2001. 144(3): p. 523-32.
3. Nielsen, N.H., et al., Allergic contact sensitization in an adult Danish population: two cross-sectional surveys eight years apart (the Copenhagen Allergy Study). Acta Derm Venereol, 2001. 81(1): p. 31-4.
4. Fonacier, L.S., S.C. Dreskin, and D.Y. Leung, Allergic skin diseases. J Allergy Clin Immunol. 125(2 Suppl 2): p. S138-49.
5. *EC 1907/2006, in Regulation (EC) No 1907/2006 of the European Parliament and of the Council of 18 December 2006 concerning the Registration, Evaluation, Authorisation and Restriction of Chemicals (REACH), establishing a European Chemicals Agency, amending Directive 1999/45/EC and repealing Council Regulation (EEC) No 793/93 and Commission Regulation (EC) No 1488/94 as well as Council Directive 76/769/EEC and Commission Directives 91/155/EEC, 93/67/EEC, 93/105/EC and 2000/21/EC.*
6. Basketter, D.A., et al., Local lymph node assay - validation, conduct and use in practice. Food Chem Toxicol, 2002. 40(5): p. 593-8.
7. Magnusson, B. and A.M. Kligman, The identification of contact allergens by animal assay. The guinea pig maximization test. J Invest Dermatol, 1969. 52(3): p. 268-76.

8. Masterson, A.J., et al., MUTZ-3, a human cell line model for the cytokine-induced differentiation of dendritic cells from CD34+ precursors. Blood, 2002. 100(2): p. 701-3.

9. Santegoets, S.J., et al., A CD34(+) human cell line model of myeloid dendritic cell differentiation: evidence for a CD14(+)CD11b(+) Langerhans cell precursor. J Leukoc Biol, 2006. 80(6): p. 1337-44.

10. Larsson, K., M. Lindstedt, and C.A. Borrebaeck, Functional and transcriptional profiling of MUTZ-3, a myeloid cell line acting as a model for dendritic cells. Immunology, 2006. 117(2): p. 156-66.

11. Rasaiyaah, J., et al., Dendritic cells and myeloid leukaemias: plasticity and commitment in cell differentiation. Br J Haematol, 2007. 138(3): p. 281-90.

12. Cortes, C. and V. Vapnik, Support-Vector Networks. Machine Learning, 1995. 20(3): p. 273-297.

13. Kimber, I., et al., Allergic contact dermatitis. Int Immunopharmacol, 2002. 2(2-3): p. 201-11.

14. Gerberick, G.F., et al., Compilation of historical local lymph node data for evaluation of skin sensitization alternative methods. Dermatitis, 2005. 16(4): p. 157-202.

15. Kimber, I., et al., Classification of contact allergens according to potency: proposals. Food Chem Toxicol, 2003. 41(12): p. 1799-809.

16. Kligman, A.M., The identification of contact allergens by human assay. 3. The maximization test: a procedure for screening and rating contact sensitizers. J Invest Dermatol, 1966. 47(5): p. 393-409.

17. Haneke, K.E., et al., ICCVAM evaluation of the murine local lymph node assay. Data analyses completed by the National Toxicology Program Interagency Center for the Evaluation of Alternative Toxicological Methods. Regul Toxicol Pharmacol, 2001. 34(3): p. 274-86.

18. dos Santos, G.G., et al., Progress on the development of human in vitro dendritic cell based assays for assessment of the sensitizing potential of a compound. Toxicol Appl Pharmacol, 2009. 236(3): p. 372-82.

19. Hooyberghs, J., et al., A cell-based in vitro alternative to identify skin sensitizers by gene expression. Toxicol Appl Pharmacol, 2008. 231(1): p. 103-11.

20. Lambrechts, N., et al., Gene markers in dendritic cells unravel pieces of the skin sensitization puzzle. Toxicol Lett, 2010. 196(2): p. 95-103.

21. Lambrechts, N., et al., Assessment of chemical skin-sensitizing potency by an in vitro assay based on human dendritic cells. Toxicol Sci, 2010. 116(1): p. 122-9.

22. Lindstedt, M., et al., Genomic and functional delineation of dendritic cells and memory T cells derived from grass pollen-allergic patients and healthy individuals. Int Immunol, 2005. 17(4): p. 401-9.

23. R Development Core Team. R: A language and environment for statistical computing. R Foundation for Statistical Computing. 2008; Available from: http://www. R-project.org.

24. R package e1071. Available from: http://cran.r-project.org/web/packages/e1071/index.html.

25. Lasko, T.A., et al., The use of receiver operating characteristic curves in biomedical informatics. J Biomed Inform, 2005. 38(5): p. 404-15.

26. Warbrick, E.V., et al., Influence of application vehicle on skin sensitization to methylchloroisothiazolinone/methylisothiazolinone: an analysis using the local lymph node assay. Contact Dermatitis, 1999. 41(6): p. 325-9.

27. Roberts, D.W., et al., Mechanistic applicability domain classification of a local lymph node assay dataset for skin sensitization. Chem Res Toxicol, 2007. 20(7): p. 1019-30.

28. Gerberick, G.F., et al., Contact allergenic potency: correlation of human and local lymph node assay data. Am J Contact Dermat, 2001. 12(3): p. 156-61.

29. Basketter, D.A., D. Sanders, and I.R. Jowsey, The skin sensitization potential of resorcinol: experience with the local lymph node assay. Contact Dermatitis, 2007. 56(4): p. 196-200.

30. Ryan, C.A., et al., Activity of human contact allergens in the murine local lymph node assay. Contact Dermatitis, 2000. 43(2): p. 95-102.

31. APPENDIX C; Comparative LLNA: BrdU-FC, Traditional LLNA, Guinea Pig Skin Sensitization, and Human Data. 2008 Available from: http://iccvam.niehs.nih.gov/methods/immunotox/fcLLNA/Appx/AppendixC_LLNA_FC07Jan08FD.pdf.

32. Patlewicz, G., et al., Skin-sensitization structure-activity relationships for aldehydes. Contact Dermatitis, 2001. 44(6): p. 331-6.

33. Basketter, D.A., G.F. Gerberick, and I. Kimber, Strategies for identifying false positive responses in predictive skin sensitization tests. Food Chem Toxicol, 1998. 36(4): p. 327-33.

34. Ashby, J., et al., Structure activity relationships in skin sensitization using the murine local lymph node assay. Toxicology, 1995. 103(3): p. 177-94.

35. Sakaguchi, H., et al., Development of an in vitro skin sensitization test using human cell lines; human Cell Line Activation Test (h-CLAT). II. An interlaboratory study of the h-CLAT. Toxicol In Vitro, 2006. 20(5): p. 774-84.

36. The Murine Local Lymph Node Assay: A Test Method for Assessing the Allergic Contact Dermatitis Potential of Chemicals/Compounds. 1999;

Available from:

http://iccvam.niehs.nih.gov/docs/immunotox_docs/IIna/IInarep.pdf.

**TABLES**

Table 1. List of sensitizing and non-sensitizing chemicals, based on murine LLNA classification, tested in the cell-based assay.

| Compound | Abbreviation | Potency | LLNA | HMT[1] | HPTA[1] |
|---|---|---|---|---|---|
| *Sensitizers* | | | | | |
| 2,4-Dinitrochlorobenzene | DNCB | Extreme [15] | + [15] | | |
| Oxazolone | OXA | Extreme [15] | + [15] | | |
| Potassium dichromate | PD | Extreme [14] | + [14] | + | + |
| Kathon CH (MC/MCI) | KCG | Extreme [14, 26] | + [14, 26] | | |
| Formaldehyde | FA | Strong [15] | + [15] | + | + |
| 2-Aminophenol | 2AP | Strong [27] | + [27] | | |
| 2-nitro-1,4-Phenylendiamine | NPDA | Strong [27] | + [27] | | |
| p-Phenylendiamine | PPD | Strong [28] | + [28] | + | + |
| Hexylcinnamic aldehyde | HCA | Moderate [15] | + [15] | | |
| 2-Hydroxyethyl acrylate | 2HA | Moderate [27] | + [27] | | + |
| 2-Mercaptobenzothiazole | MBT | Moderate [27] | + [27] | + | + |
| Glyoxal | GO | Moderate [27] | + [27] | + | |
| Cinnamaldehyde | CALD | Moderate [28] | + [28] | + | + |
| Isoeugenol | IEU | Moderate [28] | + [28] | | + |
| Ethylendiamine | EDA | Moderate [14] | + [14] | | |
| Resorcinol | RC | Moderate [29] | + [29] | - | + |
| Cinnamic alcohol | CALC | Weak [27] | + [28] | | |
| Eugenol | EU | Weak [28] | + [28] | | + |
| Penicillin G | PEN G | Weak [28] | + [28] | + | |
| Geraniol | GER | Weak [14] | + [14] | - | + |
| *Non-sensitizers* | | | | | |
| 1-Butanol | BUT | | - [30] | | |
| 4-Aminobenzoic acid | PABA | | - [31] | - | + |
| Benzaldehyde | BA | | - [32] | | |
| Chlorobenzene | CB | | - [14] | | |
| Diethyl phthalate | DP | | - [28] | | |
| Dimethyl formamide | DF | | - [26] | | |
| Ethyl vanillin | EV | | - [32] | | |
| Glycerol | GLY | | - [28] | | |
| Isopropanol | IP | | - [28] | | |

(continued)

| Non-sensitizers | | | | | |
|---|---|---|---|---|---|
| Lactic acid | LA | | - [14] | | |
| Methyl salicylate | MS | | - [14] | - | |
| Octanoic acid | OA | | - [33] | | |
| Propylene glycol | PG | | - [31] | | |
| Phenol | PHE | | - [33] | - | |
| p-Hydroxybenzoic acid | HBA | | - [34] | | |
| Potassium permanganate | PP | | - | | |
| Salicylic acid | SA | | - [14] | - | |
| Sodium dodecyl sulphate | SDS | | +[2] [14, 33] | - | |
| Tween 80 | T80 | | - [35] | | + |
| Zinc sulphate | ZS | | +[2] [36] | | |

[1] HMT, Human Maximation Test; HPTA, Human Patch Test Allergen. Information is derived from [17].
[2] False positives in LLNA.

**Table 2.** Vehicle and concentrations used for testing.

| Compound | Abbreviation | Vehicle | Max solubility (μM) | Rv90 (μM) | Concentration In culture (μM) |
|---|---|---|---|---|---|
| Sensitizers | | | | | |
| 2,4-Dinitrochlorobenzene | DNCB | DMSO | - | 4 | 4 |
| Oxazolone | OXA | DMSO | 250 | - | 250 |
| Potassium dichromate | PD | Water | 51.02 | 1.5 | 1.5 |
| Kathon CG (MC/MCI)[*] | KCG | Water | - | 0.0035% | 0.0035% |
| Formaldehyde | FA | Water | - | 80 | 80 |
| 2-Aminophenol | 2AP | DMSO | - | 100 | 100 |
| 2-nitro-1,4-Phenylendiamine | NPDA | DMSO | - | 300 | 300 |
| p-Phenylendiamine | PPD | DMSO | 566 | 75 | 75 |
| Hexylcinnamic aldehyde | HCA | DMSO | 32.34 | - | 32.24 |
| 2-Hydroxyethyl acrylate | 2HA | Water | - | 100 | 100 |
| 2-Mercaptobenzothiazole | MBT | DMSO | 250 | - | 250 |
| Glyoxal | GO | Water | - | 300 | 300 |
| Cinnamaldehyde | CALD | Water | - | 120 | 120 |
| Isoeugenol | IEU | DMSO | 641 | 300 | 300 |
| Ethylendiamine | EDA | Water | - | - | 500 |
| Resorcinol | RC | Water | - | - | 500 |
| Cinnamic alcohol | CALC | DMSO | 500 | - | 500 |
| Eugenol | EU | DMSO | 649 | 300 | 300 |
| Penicillin G | PEN G | Water | - | - | 500 |

(continued)

| Compound | Abbreviation | Vehicle | Max solubility (μM) | Rv90 (μM) | Concentration In culture (μM) |
|---|---|---|---|---|---|
| *Sensitizers* | | | | | |
| Geraniol | GER | DMSO | - | - | 500 |
| ***Non-sensitizers*** | | | | | |
| 1-Butanol | BUT | DMSO | - | - | 500 |
| 4-Aminobenzoic acid | PABA | DMSO | - | - | 500 |
| Benzaldehyde | BA | DMSO | 250 | - | 250 |
| Chlorobenzene | CB | DMSO | 98 | - | 98 |
| Diethyl phthalate | DP | DMSO | 50 | - | 50 |
| Dimethyl formamide | DF | Water | - | - | 500 |
| Ethyl vanillin | EV | DMSO | - | - | 500 |
| Glycerol | GLY | Water | - | - | 500 |
| Isopropanol | IP | Water | - | - | 500 |
| Lactic acid | LA | Water | - | - | 500 |
| Methyl salicylate | MS | DMSO | - | - | 500 |
| Octanoic acid | OA | DMSO | 504 | - | 500 |
| Peopylene glycol | PG | Water | - | - | 500 |
| Phenol | PHE | Water | - | - | 500 |
| p-Hydroxybenzoic acid | HBA | DMSO | 250 | - | 250 |
| Potassium permanganate | PP | Water | 38 | - | 38 |
| Salicylic acid | SA | DMSO | - | - | 500 |
| Sodium dodecyl sulphate | SDS | Water | - | 200 | 200 |
| Tween 80 | T80 | DMSO | - | - | 500 |
| Zinc sulphate | ZS | Water | 126 | - | 126 |
| [1] Kathon CG is a mixture of the compounds MC and MCI. The concentration of this mixture is given in %. | | | | | |

Table 3. Differentially expressed genes in MUTZ-3 cells stimulated with sensitizing chemicals as compared to non-sensitizing agents and controls.

| Gene Title | Gene Symbol | NCBI reference sequence |
|---|---|---|
| **Table 3A** | | |
| squalene epoxidase | SQLE | NM_003129 |
| taste receptor, type 2, member 5 | TAS2R5 | NM_018980 |
| keratinocyte growth factor-like protein 1/2/hypothetical protein FLJ20444 | KGFLP1/2/ FLJ20444 | AF523265 |
| transmembrane anterior posterior transformation 1 | TAPT1 | NM_153365 |
| Sprouty homolog 2 | SPRY2 | NM_005842 |
| B-cell CLL/lymphoma 7A | BCL7A | NM_020993 |
| solute carrier family 25, member 32 | SLC25A32 | NM_030780 |

(continued)

| Gene Title | Gene Symbol | NCBI reference sequence |
|---|---|---|
| **Table 3A** | | |
| ferritin, heavy polypeptide pseudogene 1 | FTHP1 | GENSCAN00000008165 |
| ATPase, H+ transporting, lysosomal 50/57kDa, V1 subunit H | ATP6V1H | NM_015941 |
| Histone cluster 1, H1e | HIST1H1E | NM_005321 |
| | | |
| **Table 3B** | | |
| 4-aminobutyrate aminotransferase | ABAT | NM_020686 |
| abhydrolase domain containing 5 | ABHD5 | NM_016006 |
| alkaline ceramidase 2 | ACER2 | NM_001010887 |
| ATP citrate lyase | ACLY | NM_001096 |
| actin-related protein 10 homolog | ACTR10 | NM_018477 |
| ADAM metallopeptidase domain 20 | ADAM20 | NM_003814 |
| Retrotransposed pseudogene AL391261.2-201 | AL391261.2-201 | GENSCAN00000063078 |
| aldehyde dehydrogenase 18 family, member A1 | ALDH18A1 | NM_002860 |
| aldehyde dehydrogenase 1 family, member B1 | ALDH1B1 | NM_000692 |
| alkB, alkylation repair homolog 6 (E. coli) | ALKBH6 | NM_032878 |
| anaphase promoting complex subunit 1 | ANAPC1 | NM_022662 |
| anaphase promoting complex subunit 5 | ANAPC5 | NM_016237 |
| ankyrin repeat, family A (RFXANK-like), 2 | ANKRA2 | NM_023039 |
| ADP-ribosylation factor GTPase activating protein 3 | ARFGAP3 | NM_014570 |
| Rho GTPase activating protein 9 | ARHGAP9 | NM_032496 |
| ankyrin repeat and SOCS box-containing 7 | ASB7 | NM_198243 |
| ATPase, H+ transporting, lysosomal 9kDa, V0 subunit e1 | ATP6V0E1 | NM_003945 |
| bridging integrator 2 | BIN2 | NM_016293 |
| bleomycin hydrolase | BLMH | NM_000386 |
| brix domain containing 1/ribosome production factor 2 homolog | BXDC1 / RPF2 | ENST00000368864 |
| chromosome 11 open reading frame 61 | C11orf61 | NM_024631 |
| chromosome 11 open reading frame 67 | C11orf67 | NM_024684 |
| chromosome 12 open reading frame 57 | C12orf57 | NM_138425 |
| chromosome 13 open reading frame 18 | C13orf18 | NM_025113 |
| chromosome 15 open reading frame 24 | C15orf24 | NM_020154 |
| chromosome 19 open reading frame 54 | C19orf54 | NM_198476 |
| chromosome 1 open reading frame 174 | C1orf174 | NM_207356 |
| chromosome 1 open reading frame 183 | C1orf183 | NM_019099 |
| chromosome 20 open reading frame 111 | C20orf111 | NM_016470 |
| chromosome 20 open reading frame 24 | C20orf24 | BC004446 |

(continued)

| Table 3B | | |
|---|---|---|
| chromosome 3 open reading frame 62 / ubiquitin specific peptidase 4 (proto-oncogene) | C3orf62 / USP4 | BC023586 |
| chromosome 9 open reading frame 89 | C9orf89 | BC038856 |
| coactivator-associated arginine methyltransferase 1 | CARM1 | NM_199141 |
| CD33 molecule | CD33 | NM_001772 |
| CD86 molecule | CD86 | NM_175862 |
| CD93 molecule | CD93 | NM_012072 |
| cytochrome c oxidase subunit VIIa polypeptide 2 like | COX7A2L | NM_004718 |
| corticotropin releasing hormone binding protein | CRHBP | NM_001882 |
| chondroitin sulfate N-acetylgalactosaminyltransferase 2 | CSGALNACT2 | NM_018590 |
| Cytochrome P450 51A1 | CYP51A1 | NM_000786.2 |
| DDRGK domain containing 1 | DDRGK1 | NM_023935 |
| DEAD (Asp-Glu-Ala-Asp) box polypeptide 21 | DDX21 | NM_004728 |
| 24-dehydrocholesterol reductase | DHCR24 | NM_014762 |
| 7-dehydrocholesterol reductase | DHCR7 | NM_001360 |
| DEAH (Asp-Glu-Ala-His) box polypeptide 33 | DHX33 | NM_020162 |
| DnaJ (Hsp40) homolog, subfamily B, member 4 | DNAJB4 | NM_007034 |
| DnaJ (Hsp40) homolog, subfamily B, member 9 | DNAJB9 | NM_012328 |
| DnaJ (Hsp40) homolog, subfamily C, member 5 | DNAJC5 | NM_025219 |
| DnaJ (Hsp40) homolog, subfamily C, member 9 | DNAJC9 | NM_015190 |
| D-tyrosyl-tRNA deacylase 1 homolog | DTD1 | NM_080820 |
| ER degradation enhancer, mannosidase alpha-like 2 | EDEM2 | NM_018217 |
| ecotropic viral integration site 2B | EVI2B | NM_006495 |
| family with sequence similarity 36, member A | FAM36A | NM_198076 |
| family with sequence similarity 86, member A | FAM86A | NM_201400 |
| Fas (TNF receptor superfamily, member 6) | FAS | NM_000043 |
| MGC44478 | FDPSL2A | NR_003262 |
| ferredoxin reductase | FDXR | NM_024417 |
| forkhead box 04 | FOXO4 | NM_005938 |
| FTHL10-001, Transcribed processed pseudogene | FTHL 10-001 | NR_002200 |
| fucosidase, alpha-L- 2, plasma | FUCA2 | NM_032020 |
| growth arrest-specific 2 like 3 | GAS2L3 | NM_174942 |
| ganglioside induced differentiation associated protein 2 | GDAP2 | NM_017686 |
| growth differentiation factor 11 | GDF11 | NM_005811 |
| glutaredoxin (thioltransferase) | GLRX | NM_002064 |
| guanine nucleotide binding protein-like 3 | GNL3L | NM_019067 |
| glucosamine-phosphate N-acetyltransferase 1 | GNPNAT1 | NM_198066 |
| glutathione reductase | GSR | NM_000637 |

(continued)

| Table 3B | | |
|---|---|---|
| GTF2I repeat domain containing 2B/2/ 2 pseudogene | GTF2IRD2B/2/2P | BC067859 |
| general transcription factor IIIC, polypeptide 2 beta | GTF3C2 | NM_001521 |
| HMG-box transcription factor 1 | HBP1 | NM_012257 |
| histone cluster 1, H1c | HIST1H1C | NM_005319 |
| histone cluster 1, H2ae | HIST1H2AE | NM_021052 |
| histone cluster 1, H2be | HIST1H2BE | NM_003523 |
| histone clusters 1, H2bm/2, H3, pseudogene 2/2, H2b/a | HIST1H2BM/ HIST2H3PS2/BF/A | NM_001024599 |
| histone cluster 1, H3g | HIST1H3G | NM_003534 |
| histone cluster 1, H3j | HIST1H3J | NM_003535 |
| histone cluster 1, H4a | HIST1H4A | NM_003538 |
| histone clusters 2, H2aa3 / 2, H2aa4 | HIST2H2AA3 /4 | NM_003516 |
| high-mobility group box 3 | HMGB3 | NM_005342 |
| 3-hydroxy-3-methylglutaryl-Coenzyme A reductase | HMGCR | NM_000859 |
| 3-hydroxy-3-methylglutaryl-Coenzyme A synthase 1 | HMGCS1 | NM_001098272 |
| heme oxygenase (decycling) 1 | HMOX1 | NM_002133 |
| heterogeneous nuclear ribonucleoprotein L | HNRNPL | NM_001533 |
| insulin receptor substrate 2 | IRS2 | NM_003749 |
| iron-sulfur cluster scaffold homolog | ISCU | NM_014301 |
| interferon stimulated exonuclease gene 20kDa-like 2 | ISG20L2 | NM_030980 |
| potassium voltage-gated channel, Isk-related family, member 3 | KCNE3 | NM_005472 |
| hypothetical protein LOC100132855/ATPase, H+ transporting, lysosomal 38kDa, V0 subunit d1 | LOC100132855/ ATP6V0D1 | NM_004691 |
| hCG1651476 | LOC284417 | NM_001085488 |
| golgi autoantigen, golgin subfamily a, 6 pseudogene / mitochondrial poly(A) polymerase | LOC729668/ MTPAP | NM 018109 |
| lysophosphatidic acid receptor 1 | LPAR1 | NM_057159 |
| leucine-rich PPR-motif containing | LRPPRC | NM_133259 |
| lymphocyte antigen 96 | LY96 | NM_015364 |
| mitogen-activated protein kinase kinase 1 | MAP2K1 | NM_002755 |
| mitogen-activated protein kinase 13 | MAPK13 | NM_002754 |
| methyltransferase like 2A | METTL2A | NM_181725 |
| Brain cDNA clone: similar to human METTL2 | METTL2B | NM_018396.1 |
| Methyltransferase like 2B | METTL2B | NM_018396.2 |
| microsomal glutathione S-transferase 3 | MGST3 | NM_004528 |
| mitochondrial ribosomal protein L30 | MRPL30 | NM_145212 |
| mitochondrial ribosomal protein L4 | MRPL4 | NM_146388 |
| mitochondrial ribosomal protein S17 | MRPS17 | NM_015969 |
| 5-methyltetrahydrofolate-homocysteine methyltransferase | MTR | NM_000254 |

(continued)

| Table 3B | | |
|---|---|---|
| MYB binding protein (P160) 1a | MYBBP1A | NM_014520 |
| neighbor of BRCA1 gene 1 | NBR1 | NM_031858 |
| nuclear import 7 homolog | NIP7 | NM_016101 |
| NLR family, pyrin domain containing 12 | NLRP12 | NM_144687 |
| nucleolar protein family 6 (RNA-associated) | NOL6 | NM_022917 |
| NAD(P)H dehydrogenase, quinone 1 | NQO1 | NM_000903 |
| nuclear receptor binding protein 1 | NRBP1 | NM_013392 |
| nucleotide binding protein-like | NUBPL | NM_025152 |
| nudix (nucleoside diphosphate linked moiety X)-type motif 14 | NUDT14 | NM_177533 |
| nuclear fragile X mental retardation protein interacting protein 1 | NUFIP1 | NM_012345 |
| nucleoporin 153kDa | NUP153 | NM_005124 |
| olfactory receptor, family 5, subfamily B, member 21 | OR5B21 | NM_001005218 |
| PAS domain containing serine/threonine kinase | PASK | NM_015148 |
| PRKC, apoptosis, WT1, regulator | PAWR | NM_002583 |
| PDGFA associated protein 1 | PDAP1 | NM_014891 |
| phosphodiesterase 1B, calmodulin-dependent | PDE1B | NM_000924 |
| phosphoribosylformylglycinamidine synthase | PFAS | NM_012393 |
| pleckstrin homology-like domain, family A, member 3 | PHLDA3 | NM_012396 |
| phosphoinositide-3-kinase adaptor protein 1 | PIK3AP1 | NM_152309 |
| PTEN induced putative kinase 1 | PINK1 | NM_032409 |
| phosphomannomutase 2 | PMM2 | NM_000303 |
| partner of NOB1 homolog | PNO1 | NM_020143 |
| polymerase (RNA) II (DNA directed) polypeptide E, 25kDa | POLR2E | NM_002695 |
| polymerase (RNA) III (DNA directed) polypeptide E (80kD) | POLR3E | NM_018119 |
| protein phosphatase 1D magnesium-dependent, delta isoform | PPM1D | BC042418 |
| phosphatidylinositol-3,4,5-trisphosphate-dependent Rac exchange factor 1 | PREX1 | NM_020820 |
| proline-serine-threonine phosphatase interacting protein 1 | PSTPIP1 | NM_003978 |
| prothymosin, alpha | PTMA | NM_016184/ 024809 |
| RAB33B, member RAS oncogene family | RAB33B | NM_031296 |
| renin binding protein | RENBP | NM_002910 |
| replication factor C (activator 1) 2, 40kDa | RFC2 | NM_181471 |
| ribonuclease H1 | RNASEH1 | NM_002936 |
| ring finger protein 146 | RNF146 | NM_030963 |
| ring finger protein 24 | RNF24 | NM_007219 |
| ring finger protein 26 | RNF26 | NM_032015 |
| Havana pseudogene RP1-274L14.2-001 | RP1-274L14.2-001 | NM_032020 |
| ribosomal protein SA / small nucleolar RNA, H/ACA box 62 | RPSA / SNORA62 | NM_014570 |

(continued)

| Table 3B | | |
|---|---|---|
| RNA pseudouridylate synthase domain containing 2 | RPUSD2 | NM_152260 |
| ribosomal RNA processing 12 homolog | RRP12 | NM_015179 |
| retinoid X receptor, alpha | RXRA | NM_002957 |
| scavenger receptor class B, member 2 | SCARB2 | NM_005506 |
| SERPINE1 mRNA binding protein 1 | SERBP1 | NM_001018067 |
| splicing factor proline/glutamine-rich | SFPQ | NM_005066 |
| solute carrier family 35, member B3 | SLC35B3 | BX538271 |
| solute carrier family 37, member 4 | SLC37A4 | NM_001467 |
| solute carrier family 5, member 6 | SLC5A6 | NM_021095 |
| sphingomyelin phosphodiesterase 4, neutral membrane | SMPD4 | NM_017751 |
| small nucleolar(sn)RNA host gene 1, non-coding/snRNA C/D box 26 | SNHG1 / SNORD26 | NM_002032 |
| small nucleolar RNA host gene 12 (non-coding) | SNHG12 | NM_207356 |
| small nucleolar RNA, H/ACA box 45 | SNORA45 | NR_002977 |
| SnRNA | SnRNA | Affymetrix: 7966223 |
| sorting nexin family member 27 | SNX27 | NM_030918 |
| sterol regulatory element binding transcription factor 2 | SREBF2 | NM_004599 |
| RRNA SSU_rRNA_5 | SSU_rRNA_5 | ENST00000386723 |
| ST3 beta-galactoside alpha-2,3-sialyltransferase 6 | ST3GAL6 | NM_006100 |
| serine/threonine kinase 17b | STK17B | NM_004226 |
| tubulin folding cofactor E-like | TBCEL | NM_152715 |
| tectonic family member 2 | TCTN2 | NM_024809 |
| toll-like receptor 6 | TLR6 | NM_006068 |
| toll-like receptor 9/twinfilin homolog 2 | TLR9 /TWF2 | NM_007284 |
| transmembrane protein 55A | TMEM55A | NM_018710 |
| transmembrane protein 59 | TMEM59 | NM_004872 |
| transmembrane protein 77 | TMEM77 | BC091509 |
| transmembrane protein 97 | TMEM97 | NM_014573 |
| tumor necrosis factor receptor superfamily, member 10c | TNFRSF10C | NM_003841 |
| translocase of outer mitochondrial membrane 34 | TOMM34 | NM_006809 |
| translocase of outer mitochondrial membrane 40 homolog | TOMM40 | BC001779 |
| translocase of outer mitochondrial membrane 5 homolog /F-box protein 10 | TOMM5 / FBXO10 | NM_012166 |
| tumor protein p53 inducible protein 3 | TP53I3 | NM_004881 |
| tumor protein p53 inducible nuclear protein 1 | TP53INP1 | NM_033285 |
| thioredoxin reductase 1 | TXNRD1 | NM_003330 |
| ubiquitin-fold modifier conjugating enzyme 1 | UFC1 | NM_016406 |
| ubiquitin specific peptidase 10 | USP10 | NM_005153 |

(continued)

| Table 3B | | |
| --- | --- | --- |
| vesicle-associated membrane protein 3 (cellubrevin) | VAMP3 | NM_004781 |
| valyl-tRNA synthetase | VARS | NM_006295 |
| vacuolar protein sorting 37 homolog A | VPS37A | NM_152415 |
| zinc finger protein 211 | ZNF211 | NM_006385 |
| zinc finger protein 223 | ZNF223 | NM_013361 |
| zinc finger protein 561 | ZNF561 | NM_152289 |
| zinc finger protein 79 | ZNF79 | NM_007135 |
| fatty acid synthase | FASN | NM_004104 |
| **Table 3 legend.** The table shows the profile genes found by t-test and Backward Elimination. Genes were annotated, using the NetAffx database from Affymetrix (www.affymetrix.com, Santa Clara USA). When found, the Unigene (www.ncbi.nlm.nih.gov/UniGene/) ID was chosen as the gene identifier. In the twelve cases where no Unigene ID was reported the best alternative ID was given. Gene names and IDs were checked against the IPA database where 189 of the 200 could be matched. In one instance only an Affymetrix ID was reported. 6 duplicate genes were removed. | | |

**Table 4.** Dominating functions in the "Prediction signature". 184 of the 200 molecules were investigated functionally using IPA. Only functions populated by 15 or more molecules were reported.

| Function | Number of molecules from signature | Molecule names | Most prominent sub functions |
| --- | --- | --- | --- |
| small molecule biochemistry | 38 | ABHD5, ACLY, ALDH18A1, BLMH, CD86, CSGALNACT2, CYP51A1, DHCR24, DHCR7, DNAJC5, FAS, FASN, FDXR, GLRX, GNPNAT1, HMGCR, HMOX1, IRS2, LPAR1, LY96, MGST3, MTR, NQO1, PASK, PDE1B, PINK1, PMM2, RENBP, RXRA, SLC25A32, SLC37A4, SLC5A6, SMPD4, SQLE, SREBF2, ST3GAL6, TLR6, TMEM55A | Metabolism (23), biosynthesis (14), modification (12), synthesis (10) |
| cell death | 33 | CD33, DDX19A, DHCR24, DNAJB9, DNAJC5, FAS, FASN, FDXR, FOXO4, GLRX, GNPNAT1, GSR, HIST1H1C, HMGB3, HMOX1, IRS2, LPAR1, MAP2K1, MAPK13, NQO1, PAWR, PDE1B, PHLDA3, PINK1, PPM1D, RXRA, SERBP1, SPRY2, STK17B, TLR6, TNFRSF10C, TP53INP1, TXNRD1 | Apoptosis (30), cell death (13) |
| lipid metabolism | 24 | ABHD5, ACLY, CYP51A1, DHCR24, DHCR7, FAS, FASN, FDXR, HMGCR, HMOX1, IRS2, LPAR1, LY96, MGST3, PASK, RENBP, RXRA, SLC37A4, SMPD4, SQLE, SREBF2, ST3GAL6, TLR6, TMEM55A | Metabolism (17), modification (11), synthesis (10) |
| hematological system development | 19 | CARM1, CD33, CD86, FAS, FOXO4, HIST1H1C, HMGB3, HMGCR, HMOX1, IRS2, LY96, NBR1, NQO1, PAWR, PIK3AP1, PPM1D, STK17B, TP53INP1, VAMP3 | Proliferation (10), apoptosis (5) |
| cellular growth and proliferation | 16 | CARM1, CD33, CD86, FAS, FOXO4, HIST1H1C, HMGB3, HMGCR, HMOX1, IRS2, LY96, NBR1, NQO1, PAWR, PIK3AP1, PPM1D, STK17B, TP53INP1, VAMP3 | Proliferation (16), growth (4) |
| molecular transport | 15 | CARM1, CD33, CD86, FAS, FOXO4, HIST1H1C, HMGB3, HMGCR, HMOX1, IRS2, LY96, NBR1, NQO1, PAWR, PIK3AP1, PPM1D, STK17B, TP53INP1, VAMP3 | Accumulation (8), quantity (5) |

(continued)

| Function | Number of molecules from signature | Molecule names | Most prominent sub functions |
|---|---|---|---|
| cell cycle | 15 | DNAJB4, DTD1, FAS, FASN, FOXO4, GDF11, HBP1, HMOX1, IRS2, MAP2K1, PAWR, PPM1D, SFPQ, SPRY2, TP531NP1 | Cell cycle progression (13), cell division (5) |
| carbohydrate metabolism | 15 | DNAJB4, DTD1, FAS, FASN, FOXO4, GDF11, HBP1, HMOX1, IRS2, MAP2K1, PAWR, PPM1D, SFPQ, SPRY2, TP531NP1 | Metabolism (9), biosynthesis (5) |

**Table 5.** Support Vector Machine (SVM) algorithm

```
filnamnTraining<-"training set.txt"
filnamnTest<-"test set.txt"
lista <- read.delim("biomarker signature.txt",header=FALSE) # EN FIL
MED DE ANALYTER

lista <- as.character(lista[[1]])
listaBoolean <- is.element(ProteinNames, lista)

group1<- "pos"
group2<- "neg"

rawfile <- read.delim(filnamnTraining)
samplenames <- as.character(rawfile[,1])
groupsTraining <- rawfile[,2]
dataTraining <- t(rawfile[,-c(1,2)])
ProteinNames <- read.delim(filnamnTraining,header=FALSE)
ProteinNames <- as.character(as.matrix(ProteinNames)[1,])
ProteinNames <- ProteinNames[-(1:2)]

listaBoolean <- is.element(ProteinNames, lista)

rownames(dataTraining) <- ProteinNames
```

```
colnames(dataTraining) <- samplenames
logdataTraining <- dataTraining
logdataTraining <- logdataTraining[listaBoolean,]

rawfile <- read.delim(filnamnTest)
samplenames <- as.character(rawfile[,1])
groupsTest <- rawfile[,2]
dataTest <- t(rawfile[,-c(1,2)])
ProteinNames <- read.delim(filnamnTest,header=FALSE)
ProteinNames <- as.character(as.matrix(ProteinNames)[1,])
ProteinNames <- ProteinNames[-(1:2)]
rownames(dataTest) <- ProteinNames
colnames(dataTest) <- samplenames
logdataTest<-dataTest
logdataTest <- logdataTest[listaBoolean,]

svmfacTraining<-  factor(rep('rest',ncol(logdataTraining)),levels=c(group1,
group2, 'rest'))
subset1Training<- is.element(groupsTraining ,
strsplit(group1,",")[[1]])
subset2Training<- is.element(groupsTraining ,
strsplit(group2,",")[[1]])
svmfacTraining[subset1Training] <- group1
svmfacTraining[subset2Training] <- group2
facTraining <-factor(as.character(svmfacTraining
[subset1Training|subset2Training]),levels=c(group1,group2))

svmfacTest<-  factor(rep('rest',ncol(logdataTest)),levels=c(group1, group2,
'rest'))
subset1Test<- is.element(groupsTest , strsplit(group1,",")[[1]])
subset2Test<- is.element(groupsTest , strsplit(group2,",")[[1]])
svmfacTest[subset1Test] <- group1
svmfacTest[subset2Test] <- group2
facTest <-factor(as.character(svmfacTest
[subset1Test|subset2Test]),levels=c(group1,group2))
 n1 <- sum(facTest ==levels(facTest )[1])
 n2 <- sum(facTest ==levels(facTest )[2])
 nsamples <- n1+n2
SampleInformation <- paste(levels(facTest )[1]," ",n1," , ",levels(facTest
)[2]," ",n2,sep="")

svmtrain <- svm(t(logdataTraining) , facTraining , kernel="linear" )
pred<-predict(svmtrain , t(logdataTest) , decision.values=TRUE)
res<-attr(pred, "decision.values")
names <- colnames(logdataTest, do.NULL=FALSE)
orden <- order(res , decreasing=TRUE)
Samples <- data.frame(names[orden],res[orden],facTest[orden])
ROCdata <- myROC(res,facTest)
SenSpe <- SensitivitySpecificity(res,facTest)

ROCplot(list(SampleInformation=SampleInformation,ROCarea=ROCdata[1],p
.value=ROCdata[2],SenSpe <- SenSpe,samples=Samples),
sensspecnumber=4)

#rows in blue are needed only for ROC evaluation.
#to assess an unknown sample, print res.
#(vector with prediction values)
```

**Claims**

1. An *in vitro* method for identifying agents capable of inducing sensitization of mammalian skin wherein the agents are capable of inducing and triggering a Type IV delayed-type hypersensitivity reaction in a mammal comprising or consisting of the steps of:

> a) exposing a population of dendritic cells or a population of dendritic-like cells to a test agent; and
> b) measuring in the cells the expression of two or more biomarker(s) selected from the group defined in Table 3A;

wherein the expression in the cells of the two or more biomarkers measured in step (b) is indicative of the sensitizing effect of the test agent.

2. The method according to Claim 1 further comprising exposing a separate population of the dendritic cells or dendritic-like cells to a negative control agent that does not sensitize human skin and measuring in the cells the expression of the two or more biomarker(s) measured in step (b) and/or exposing a separate population of the dendritic cells or dendritic-like cells to a positive control agent that sensitizes human skin and measuring in the cells the expression of the two or more biomarker(s) measured in step (b).

3. The method according to any one or the preceding claims wherein step (b) comprises measuring the expression of at least one biomarker selected from the group consisting of:

> i) taste receptor, type 2, member 5 (TAS2R5),
> ii) keratinocyte growth factor-like protein 1/2/hypothetical protein FLJ20444 (KGFLP1/2/ FLJ20444),
> iii) transmembrane anterior posterior transformation 1 (TAPT1),
> iv) sprouty homolog 2 (SPRY2),
> v) fatty acid synthase (FASN),
> vi) B-cell CLL/lymphoma 7A (BCL7A),
> vii) solute carrier family 25, member 32 (SLC25A32),
> viii) ferritin, heavy polypeptide pseudogene 1 (FTHP1),
> ix) ATPase, H+ transporting, lysosomal 50/57kDa, V1 subunit H (ATP6V1H),
> x) squalene epoxidase (SQLE), and
> xi) histone cluster 1, H1e (HIST1H1E).

4. The method according to any one of the preceding claims wherein step (b) comprises or consists of measuring the expression of at least 2 biomarkers from Table 3A, for example, at least 3, 4, 5, 6, 7, 8, 9 or 10 biomarkers from Table 3A.

5. The method according to any one of the preceding claims wherein step (b) comprises or consists of measuring the expression of at least 2 biomarkers from Table 3B, for example, at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123,124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183 or at least 184 biomarkers from Table 3B.

6. The method according to any one of the preceding claims wherein step (b) comprises measuring the expression of a nucleic acid molecule encoding the two or more biomarker(s).

7. The method according to any one of the preceding claims wherein measuring the expression of the two or more biomarker(s) in step (b) is performed using two or more binding moieties, each capable of binding selectively to a nucleic acid molecule encoding one of the biomarkers identified in Table 3A.

8. The method according to any one of Claims 1 to 5 wherein step (b) comprises measuring the expression of the protein of the two or more biomarker(s).

9. The method according to Claim 8 wherein measuring the expression of the two or more biomarker(s) in step (b) is performed using two or more binding moieties each capable of binding selectively to one of the biomarkers identified in Table 3A.

10. The method according to any one of the preceding claims wherein step (b) is performed using an array.

11. The method according to any one of the preceding claims wherein the population of dendritic cells or population of dendritic-like cells is a population of dendritic-like cells.

12. The method according to Claim 11 wherein the dendritic-like cells are myeloid dendritic-like cells selected from the group consisting of KG-1, THP-1, U-937, HL-60, Monomac-6, AML-193 and MUTZ-3.

13. An array for use in a method according any one of the preceding claims, wherein the binding moieties of the array consist of binding moieties for between 10 and 194 of the biomarkers defined in Table 3, and wherein the binding moieties of the array comprise two or more binding moieties as defined in Claim 7 and/or Claim 9.

14. Use of two or more biomarkers selected from the group defined in Table 3A in combination for identifying hypersensitivity response sensitising agents.

15. An analytical kit for use in a method according any one of Claims 1 to 14 comprising:

A) an array according to Claim 13; and
B) instructions for performing the method as defined in any one of Claims 1 to 14.

**Patentansprüche**

1. *In-vitro*-Verfahren zum Identifizieren von Mitteln, die in der Lage sind, Säugerhaut zu sensibilisieren, wobei die Mittel in der Lage sind, eine Typ-IV-verzögerte Hypersensibilitätsreaktion in einem Säuger hervorzurufen, die folgenden Schritte umfassend oder daraus bestehend:

a) Aussetzen einer Population aus dendritischen Zellen oder einer Population aus Zellen, die dendritischen Zellen ähneln, einem Testmittel; und
b) Messen der Exprimierung von zwei oder mehr Biomarkern, ausgewählt aus der in Tabelle 3A definierten Gruppe, in den Zellen;

wobei die in Schritt (b) gemessene Exprimierung der zwei oder mehr Biomarker in den Zellen auf die sensibilisierende Wirkung des Testmittels hindeutet.

2. Verfahren nach Anspruch 1, ferner umfassend das Aussetzen einer separaten Population aus den dendritischen Zellen oder aus den Zellen, die dendritischen Zellen ähneln, einem negativen Kontrollmittel, welches menschliche Haut nicht sensibilisiert, und Messen der Exprimierung der zwei oder mehr in Schritt (b) gemessenen Biomarker in den Zellen und/oder Aussetzen einer separaten Population aus den dendritischen Zellen oder den Zellen, die dendritischen Zellen ähneln, einem positiven Kontrollmittel, welches menschliche Haut sensibilisiert, und Messen der Exprimierung der zwei oder mehr in Schritt (b) gemessenen Biomarker in den Zellen.

3. Verfahren nach einem der vorhergehenden Ansprüche, wobei Schritt (b) das Messen der Exprimierung von wenigstens einem Biomarker umfasst, ausgewählt aus der Gruppe bestehend aus:

i) Geschmacksrezeptor, Typ 2, Mitglied 5 (TAS2R5),
ii) Keratinozytenwachstumsfaktor-ähnlichem Protein 1/2/hypothetischem Protein FLJ20444 (KGFLP1/2/FLJ20444),
iii) Transmembraner anterior-posterior-Transformation 1 (TAPT1),
iv) Sprouty-Homolog 2 (SPRY2),
v) Fettsäure-Synthase (FASN),
vi) B-Zell CLL/Lymphom 7A (BCL7A),
vii) SLC-Transporter Familie 25, Mitglied 32 (SLC25A32),
viii) Ferritin, schwerem Polypeptid-Pseudogen 1 (FTHP1),
ix) ATPase, H+-transportierendem, lysosomalem 50/57 kDa, V1 Untereinheit H (ATP6V1 H),

x) Squalenepoxidase (SQLE), und

xi) Histoncluster 1, H1e (HIST1H1E).

4. Verfahren nach einem der vorhergehenden Ansprüche, wobei Schritt (b) das Messen der Exprimierung von wenigstens 2 Biomarkern aus Tabelle 3A umfasst oder daraus besteht, wie zum Beispiel wenigstens 3, 4, 5, 6, 7, 8, 9 oder 10 Biomarkern aus Tabelle 3A.

5. Verfahren nach einem der vorhergehenden Ansprüche, wobei Schritt (b) das Messen der Exprimierung von wenigstens 2 Biomarkern aus Tabelle 3B, zum Beispiel wenigstens 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183 oder wenigstens 184 Biomarkern aus Tabelle 3B umfasst oder daraus besteht.

6. Verfahren nach einem der vorhergehenden Ansprüche, wobei Schritt (b) das Messen der Exprimierung eines Nukleinsäuremoleküls, welches für die zwei oder mehr Biomarker codiert, umfasst.

7. Verfahren nach einem der vorhergehenden Ansprüche, wobei das Messen der Exprimierung der zwei oder mehr Biomarker in Schritt (b) unter Einsatz von zwei oder mehr Bindungsmolekülteilen durchgeführt wird, die alle in der Lage sind, selektiv an ein Nukleinsäuremolekül, das einen der in Tabelle 3A identifizierten Biomarker kodiert, zu binden.

8. Verfahren nach einem der Ansprüche 1 bis 5, wobei Schritt (b) das Messen der Exprimierung des Proteins der zwei oder mehr Biomarker umfasst.

9. Verfahren nach Anspruch 8, wobei das Messen der Exprimierung der zwei oder mehr Biomarker in Schritt (b) unter Einsatz von zwei oder mehr Bindungsmolekülteilen durchgeführt wird, die alle in der Lage sind, selektiv an einen der in Tabelle 3A identifizierten Biomarker zu binden.

10. Verfahren nach einem der vorhergehenden Ansprüche, wobei Schritt (b) unter Einsatz eines Arrays durchgeführt wird.

11. Verfahren nach einem der vorhergehenden Ansprüche, wobei die Population aus dendritischen Zellen oder die Population aus Zellen, die dendritischen Zellen ähneln, eine Population aus Zellen ist, die dendritischen Zellen ähneln.

12. Verfahren nach Anspruch 11, wobei die Zellen, die dendritischen Zellen ähneln, myeloische Zellen sind, die dendritischen Zellen ähneln, ausgewählt aus der Gruppe bestehend aus KG-1, THP-1, U-937, HL-60, Monomac-6, AML-193 und MUTZ-3.

13. Array für den Gebrauch nach einem der vorhergehenden Ansprüche, wobei die Bindungsmolekülteile des Arrays aus Bindungsmolekülteilen für zwischen 10 und 194 der in Tabelle 3 definierten Biomarker bestehen und wobei die Bindungsmolekülteile des Arrays zwei oder mehr Bindungsmolekülteile nach Anspruch 7 und/oder 9 umfassen.

14. Gebrauch von zwei oder mehr Biomarkern, ausgewählt aus der in Tabelle 3A definierten Gruppe, in Kombination zum Identifizieren von für eine Hypersensibilitätsreaktion sensibilisierenden Mitteln.

15. Analysekit für den Gebrauch in einem Verfahren nach einem der Ansprüche 1 bis 14, Folgendes umfassend:

A) ein Array nach Anspruch 13; und
B) Anweisungen zum Durchführen des Verfahrens nach einem der Ansprüche 1 bis 14.

**Revendications**

1. Procédé *in vitro* d'identification d'agents capables d'induire une sensibilisation de la peau d'un mammifère, dans lequel les agents sont capables d'induire et de déclencher une réaction d'hypersensibilité retardée de type IV chez un mammifère, comprenant ou étant constitué des étapes consistant à :

   a) exposer une population de cellules dendritiques ou une population de cellules apparentées aux cellules dendritiques à un agent d'essai ; et
   b) mesurer dans les cellules l'expression de deux biomarqueurs ou plus choisis dans le groupe défini dans le Tableau 3A ;

   dans lequel l'expression dans les cellules de deux biomarqueurs ou plus mesurée dans l'étape (b) indique l'effet de sensibilisation de l'agent d'essai.

2. Procédé selon la revendication 1, comprenant en outre l'exposition d'une population distincte de cellules dendritiques ou de cellules apparentées aux cellules dendritiques à un agent de contrôle négatif qui ne sensibilise pas la peau d'un être humain et la mesure dans les cellules de l'expression de deux biomarqueurs ou plus mesurée dans l'étape (b) et/ou l'exposition d'une population distincte de cellules dendritiques ou de cellules apparentées aux cellules dendritiques à un agent de contrôle positif qui sensibilise la peau d'un être humain et la mesure dans les cellules de l'expression de deux biomarqueurs ou plus mesurée dans l'étape (b).

3. Procédé selon l'une quelconque des revendications précédentes, dans lequel l'étape (b) comprend la mesure de l'expression d'au moins un biomarqueur choisi dans le groupe constitué par :

   i) le récepteur du goût, type 2, élément 5 (TAS2R5),
   ii) la protéine apparentée au facteur de croissance des kératinocytes 1/2/protéine hypothétique FLJ20444 (KGFLP1/2/FLJ20444),
   iii) la transformation transmembranaire antérieure et postérieure 1 (TAPT1),
   iv) l'homologue sprouty 2 (SPRY2),
   v) l'acide gras synthase (FASN),
   vi) la LLC à cellules B/le lymphome 7A (BCL7A),
   vii) la famille des transporteurs de solutés 25, élément 32 (SLC25A32),
   viii) le pseudogène ferritine du polypeptide lourd 1 (FTHP1),
   ix) l'ATPase, transportant H+, lysosomale 50/57 kDa, sous-unité H de V1 (ATP6V1H),
   x) la squalène époxydase (SQLE), et
   xi) l'agrégat histone 1, H1e(HIST1H1E).

4. Procédé selon l'une quelconque des revendications précédentes, dans lequel l'étape (b) comprend ou est constituée de la mesure de l'expression d'au moins 2 biomarqueurs du Tableau 3A, par exemple, au moins 3, 4, 5, 6, 7, 8, 9 ou 10 biomarqueurs du Tableau 3A.

5. Procédé selon l'une quelconque des revendications précédentes, dans lequel l'étape (b) comprend ou est constituée de la mesure de l'expression d'au moins 2 biomarqueurs du Tableau 3A, par exemple, au moins 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183 ou au moins 184 biomarqueur du Tableau 3A.

6. Procédé selon l'une quelconque des revendications précédentes, dans lequel l'étape (b) comprend la mesure de l'expression d'une molécule d'acide nucléique codant pour deux biomarqueurs ou plus.

7. Procédé selon l'une quelconque des revendications précédentes, dans lequel la mesure de l'expression des deux biomarqueurs ou plus dans l'étape (b) est effectuée en utilisant deux fragments de liaison ou plus, chacun capable de se lier sélectivement à une molécule d'acide nucléique codant pour l'un des biomarqueurs identifiés dans le Tableau 3A.

8. Procédé selon l'une quelconque des revendications 1 à 5, dans lequel l'étape (b) comprend la mesure de l'expression de la protéine des deux biomarqueurs ou plus.

9. Procédé selon la revendication 8, dans lequel la mesure de l'expression des deux biomarqueurs ou plus dans l'étape (b) est effectuée en utilisant deux fragments de liaison ou plus, chacun capable de se lier sélectivement à l'un des biomarqueurs identifiés dans le Tableau 3A.

10. Procédé selon l'une quelconque des revendications précédentes, dans lequel l'étape (b) est effectuée à l'aide d'une matrice.

11. Procédé selon l'une quelconque des revendications précédentes, dans lequel la population de cellules dendritiques ou la population de cellules apparentées aux cellules dendritiques est une population de cellules apparentées aux cellules dendritiques.

12. Procédé selon la revendication 11, dans lequel les cellules apparentées aux cellules dendritiques sont des cellules myéloïdes apparentées aux cellules dendritiques choisies dans le groupe constitué par KG-1, THP-1, U-937, HL-60, Monomac-6, AML-193 et MUTZ-3.

13. Matrice pour une utilisation dans un procédé selon l'une quelconque des revendications précédentes, dans laquelle les fragments de liaison de la matrice sont constitués de fragments de liaison pour entre 10 et 194 des biomarqueurs définis dans le Tableau 3, et dans laquelle les fragments de liaison de la matrice comprennent deux fragments de liaison ou plus tels que définis dans la revendication 7 et/ou la revendication 9.

14. Utilisation combinée de deux biomarqueurs ou plus choisis dans le groupe défini dans le Tableau 3A pour identifier des agents de sensibilisation à une réaction d'hypersensibilité.

15. Kit analytique pour une utilisation dans un procédé selon l'une quelconque des revendications 1 à 14 comprenant :

   A) une matrice selon la revendication 13 ; et
   B) des instructions pour effectuer le procédé tel que défini dans l'une quelconque des revendications 1 à 14.

Figure 1

Figure 2A

Figure 2B

Figure 3A

Figure 3B

Figure 3C

P = 7.0e-21
Q = 1.8e-19

Figure 3D

P = 7.0e-21
Q = 1.8e-19

Non-sensitizers
Sensitizers

BUT
2AP
2HA
NPDA
BA
CB
CALC
DMSO
DNCB
DP
DF
EV
EDA
EU
FA
GER
GLY
GO
HCA
IEU
IP
KCG
LA
MBT
MS
OA
PABA
PPD
PEN G
PHE
PD
PP
PG
RC
SDS
SA
T80
ZS
dH2O
HBA0

Figure 4A

Figure 4B

Figure 4C

Figure 5

EP 2 633 077 B1

Figure 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010031799 A
- WO 2009148669 A
- US 4376110 A, David
- US 4486530 A
- EP 39578 A
- US 5856090 A
- WO 9837186 A

### Non-patent literature cited in the description

- **SZAMEIT S et al.** *Clinical & Experimental Allergy,* 2009, vol. 39, 856-868
- **CLUZEL-TAILHARDAT M et al.** *Toxicology Letters,* 2007, vol. 174, 98-108
- **LAMBRECHTS N et al.** *Toxicology and Applied Pharmacology,* 2009, vol. 236, 221-230
- **ERNARD F-X et al.** *Experimental Dermatology,* 2002, vol. 11, 59-74
- **PYTHON F.** *Toxicology and Applied Pharmacology,* 2009, vol. 239, 273-283
- **SCHOETERS E et al.** *Molecular Immunology,* 2007, vol. 44, 3222-3233
- **JOHANSSON H et al.** *BMC GENOMICS,* 2011, vol. 12, 399
- **BASKETTER, D.A. et al.** Local lymph node assay - validation, conduct and use in practice. *Food Chem Toxicol,* 2002, vol. 40 (5), 593-8
- **MAGNUSSON, B. ; A.M. KLIGMAN.** The identification of contact allergens by animal assay. The guinea pig maximization test. *J Invest Dermatol,* 1969, vol. 52 (3), 268-76
- **STEINMAN et al.** *Ann. Rev. Immunol.,* 1991, vol. 9, 271
- **FRAKER et al.** *Biochem. Biophys. Res. Comm.,* 1978, vol. 80, 49-57
- **J-F CHATAL.** Monoclonal Antibodies in Immunoscintigraphy. CRC Press, 1989
- **WINTER ; MILSTEIN.** *Nature,* 1991, vol. 349, 293-299
- **COLLETT et al.** *Methods,* 2005, vol. 37, 4-15
- **CLACKSON et al.** *Nature,* 1991, vol. 352, 624-628
- **MARKS et al.** *J Mol Biol,* 1991, vol. 222 (3), 581-97
- **SMITH.** *Science,* 1985, vol. 228 (4705), 1315-7
- **SANTI et al.** *J Mol Biol,* 2000, vol. 296 (2), 497-508
- **GUNNERIUSSON et al.** *Appl Environ Microbiol,* 1999, vol. 65 (9), 4134-40
- **KENAN et al.** *Methods Mol Biol,* 1999, vol. 118, 217-31
- **KIEKE et al.** *Proc Natl Acad Sci USA,* 1999, vol. 96 (10), 5651-6
- **HANES ; PLUCKTHUN.** *Proc Natl Acad Sci USA,* 1997, vol. 94 (10), 4937-42
- **HE ; TAUSSIG.** *Nucleic Acids Res,* 1997, vol. 25 (24), 5132-4
- **NEMOTO et al.** *FEBS Lett,* 1997, vol. 414 (2), 405-8
- **DAUGHERTY et al.** *Protein Eng,* 1998, vol. 11 (9), 825-32
- **DAUGHERTY et al.** *Protein Eng,* 1999, vol. 12 (7), 613-21
- **SHUSTA et al.** *J Mol Biol,* 1999, vol. 292 (5), 949-56
- **MORRISON et al.** *Proc. Natl. Acad. Sci. USA,* 1984, vol. 81, 6851-6855
- **BETTER et al.** *Science,* 1988, vol. 240, 1041
- **SKERRA et al.** *Science,* 1988, vol. 240, 1038
- **BIRD et al.** *Science,* 1988, vol. 242, 423
- **HUSTON et al.** *Proc. Natl. Acad. Sci. USA,* 1988, vol. 85, 5879
- **WARD et al.** *Nature,* 1989, vol. 341, 544
- **H ZOLA.** Monoclonal Antibodies: A manual of techniques. CRC Press, 1988
- **J G R HURRELL.** Monoclonal Hybridoma Antibodies: Techniques and applications. CRC Press, 1982
- **BURGES.** *Data Mining and Knowledge Discovery,* 1998, vol. 2, 121-167
- **JENKINS, R.E. ; PENNINGTON, S.R.** *Proteomics,* 2001, vol. 2, 13-29
- **LAL et al.** *Drug Discov Today,* 2002, vol. 15 (7), S143-9
- **AKHAVAN, A. ; S.R. COHEN.** The relationship between atopic dermatitis and contact dermatitis. *Clin Dermatol,* 2003, vol. 21 (2), 158-62

- **MORTZ, C.G. et al.** Prevalence of atopic dermatitis, asthma, allergic rhinitis, and hand and contact dermatitis in adolescents. The Odense Adolescence Cohort Study on Atopic Diseases and Dermatitis. *Br J Dermatol,* 2001, vol. 144 (3), 523-32
- **NIELSEN, N.H. et al.** Allergic contact sensitization in an adult Danish population: two cross-sectional surveys eight years apart (the Copenhagen Allergy Study). *Acta Derm Venereol,* 2001, vol. 81 (1), 31-4
- **FONACIER, L.S. ; S.C. DRESKIN ; D.Y. LEUNG.** Allergic skin diseases. *J Allergy Clin Immunol.,* vol. 125 (2), S138-49
- **MASTERSON, A.J. et al.** MUTZ-3, a human cell line model for the cytokine-induced differentiation of dendritic cells from CD34+ precursors. *Blood,* 2002, vol. 100 (2), 701-3
- **SANTEGOETS, S.J. et al.** A CD34(+) human cell line model of myeloid dendritic cell differentiation: evidence for a CD14(+)CD11b(+) Langerhans cell precursor. *J Leukoc Biol,* 2006, vol. 80 (6), 1337-44
- **LARSSON, K. ; M. LINDSTEDT ; C.A. BORREBAECK.** Functional and transcriptional profiling of MUTZ-3, a myeloid cell line acting as a model for dendritic cells. *Immunology,* 2006, vol. 117 (2), 156-66
- **RASAIYAAH, J. et al.** Dendritic cells and myeloid leukaemias: plasticity and commitment in cell differentiation. *Br J Haematol,* 2007, vol. 138 (3), 281-90
- **CORTES, C. ; V. VAPNIK.** upport-Vector Networks. *Machine Learning,* 1995, vol. 20 (3), 273-297
- **KIMBER, I. et al.** Allergic contact dermatitis. *Int Immunopharmacol,* 2002, vol. 2 (2-3), 201-11
- **GERBERICK, G.F. et al.** Compilation of historical local lymph node data for evaluation of skin sensitization alternative methods. *Dermatitis,* 2005, vol. 16 (4), 157-202
- **KIMBER, I. et al.** Classification of contact allergens according to potency: proposals. *Food Chem Toxicol,* 2003, vol. 41 (12), 1799-809
- **KLIGMAN, A.M.** The identification of contact allergens by human assay. 3. The maximization test: a procedure for screening and rating contact sensitizers. *J Invest Dermatol,* 1966, vol. 47 (5), 393-409
- **HANEKE, K.E. et al.** ICCVAM evaluation of the murine local lymph node assay. Data analyses completed by the National Toxicology Program Interagency Center for the Evaluation of Alternative Toxicological Methods. *Regul Toxicol Pharmacol,* 2001, vol. 34 (3), 274-86
- **DOS SANTOS, G.G. et al.** Progress on the development of human in vitro dendritic cell based assays for assessment of the sensitizing potential of a compound. *Toxicol Appl Pharmacol,* 2009, vol. 236 (3), 372-82
- **HOOYBERGHS, J. et al.** A cell-based in vitro alternative to identify skin sensitizers by gene expression. *Toxicol Appl Pharmacol,* 2008, vol. 231 (1), 103-11
- **LAMBRECHTS, N. et al.** Gene markers in dendritic cells unravel pieces of the skin sensitization puzzle. *Toxicol Lett,* 2010, vol. 196 (2), 95-103
- **LAMBRECHTS, N. et al.** Assessment of chemical skin-sensitizing potency by an in vitro assay based on human dendritic cells. *Toxicol Sci,* 2010, vol. 116 (1), 122-9
- **LINDSTEDT, M. et al.** Genomic and functional delineation of dendritic cells and memory T cells derived from grass pollen-allergic patients and healthy individuals. *Int Immunol,* 2005, vol. 17 (4), 401-9
- *R Development Core Team. R: A language and environment for statistical computing. R Foundation for Statistical Computing,* 2008, http://www. R-project.org
- *R package e1071, http://cran.r-project.org/web/packages/e1071/index.html*
- **LASKO, T.A. et al.** The use of receiver operating characteristic curves in biomedical informatics. *J Biomed Inform,* 2005, vol. 38 (5), 404-15
- **WARBRICK, E.V. et al.** Influence of application vehicle on skin sensitization to methylchloroisothiazolinone/methylisothiazolinone: an analysis using the local lymph node assay. *Contact Dermatitis,* 1999, vol. 41 (6), 325-9
- **ROBERTS, D.W. et al.** Mechanistic applicability domain classification of a local lymph node assay dataset for skin sensitization. *Chem Res Toxicol,* 2007, vol. 20 (7), 1019-30
- **GERBERICK, G.F. et al.** Contact allergenic potency: correlation of human and local lymph node assay data. *Am J Contact Dermat,* 2001, vol. 12 (3), 156-61
- **BASKETTER, D.A. ; D. SANDERS ; I.R. JOWSEY.** The skin sensitization potential of resorcinol: experience with the local lymph node assay. *Contact Dermatitis,* 2007, vol. 56 (4), 196-200
- **RYAN, C.A. et al.** Activity of human contact allergens in the murine local lymph node assay. *Contact Dermatitis,* 2000, vol. 43 (2), 95-102
- APPENDIX C; Comparative LLNA: BrdU-FC, Traditional LLNA, Guinea Pig Skin Sensitization. *Human Data,* 2008, http://iccvam.niehs.nih.gov/methods/immunotox/fcLLNA/Appx/AppendixC_LLNA_FC07Jan08FD.pdf
- **PATLEWICZ, G. et al.** Skin-sensitization structure-activity relationships for aldehydes. *Contact Dermatitis,* 2001, vol. 44 (6), 331-6
- **BASKETTER, D.A. ; G.F. GERBERICK ; I. KIMBER.** Strategies for identifying false positive responses in predictive skin sensitization tests. *Food Chem Toxicol,* 1998, vol. 36 (4), 327-33

- **ASHBY, J. et al.** Structure activity relationships in skin sensitization using the murine local lymph node assay. *Toxicology,* 1995, vol. 103 (3), 177-94
- **SAKAGUCHI, H. et al.** Development of an in vitro skin sensitization test using human cell lines; human Cell Line Activation Test (h-CLAT). II. An interlaboratory study of the h-CLAT. *Toxicol In Vitro,* 2006, vol. 20 (5), 774-84
- *The Murine Local Lymph Node Assay: A Test Method for Assessing the Allergic Contact Dermatitis Potential of Chemicals/Compounds,* 1999